# NOUVEAU FASCICULE DE BREVET EUROPEEN

(11) **EP 1 383 796 B2**
(45) Date de publication et mention de la décision concernant l'opposition: **22.08.2012**
(45) Mention de la délivrance du brevet: 15.04.2009
(21) Numéro de dépôt: 02759815.0
(22) Date de dépôt: 04.04.2002
(51) Int. Cl.: C12N 7/00

(54) **SOUCHE NEUROVIRULENTE DU VIRUS WEST-NILE ET SES APPLICATIONS**
NEUROVIRULENTER STAMM DES WEST-NILE-VIRUS UND ANWENDUNGEN DAVON
NEUROVIRULENT STRAIN OF THE WEST NILE VIRUS AND APPLICATIONS THEREOF

(30) Priorité: 04.04.2001 FR 0104599; 06.09.2001 FR 0111525
(43) Date de publication de la demande: 28.01.2004
(73) Titulaire: INSTITUT PASTEUR, 75724 Paris Cédex 15 (FR); Kimron Veterinary Institute, 50250 Beit Dagan (IL)
(72) Inventeur: DESPRES, Philippe, F-92250 La Garenne-colombes (FR); DEUBEL, Vincent, F-92000 Vanves (FR); GUENET, Jean-Louis, F-91160 Longjumeau (FR); DROUET, Marie-Thérèse, Institut Pasteur, F-75724 Paris Cedex 15 (FR); MALKINSON, Mertyn, Kimron Veterinary Institute, 50250 Beit Dagan (IL); BANET, Caroline, Kimron Veterinary Institute, 50250 Beit Dagan (IL); FRENKIEL, Marie-Pascale, F-92300 Levallois (FR); COURAGEOT, Marie-Pierre, F-75015 Paris (FR); COULIBALY, Fasséli, F-75014 Paris (FR); CATTEAU, Adeline, F-91600 Savigny Sur Orge (FR); FLAMAND, Marie, F-75013 Paris (FR); WEBER, Patrick, F-93170 Montreuil (FR); CECCALDI, Pierre-Emmanuel, F-77350 Boissise-la-bertrand (FR)
(74) Mandataire: Vialle-Presles, Marie José
(86) Numéro de dépôt international: PCT/FR2002/001168
(87) Numéro de publication internationale: WO 2002/081511

(56) Documents cités:
- JIA X-Y X-Y ET AL: "Genetic analysis of West Nile New York 1999 encephalitis virus" LANCET, XX, XX, vol. 354, no. 9194, 4 décembre 1999 (1999-12-04), pages 1971-1972, XP004262933 ISSN: 0140-6736
- LANCIOTTI R S ET AL: "Origin of the West Nile virus responsible for an outbreak of encephalitis in the northeastern United States." SCIENCE (WASHINGTON D C), vol. 286, no. 5448, 17 décembre 1999 (1999-12-17), pages 2333-2337, XP002191029 ISSN: 0036-8075
- ANDERSON JOHN F ET AL: "Isolation of West Nile virus from mosquitoes, crows, and a Cooper's hawk in Connecticut." SCIENCE (WASHINGTON D C), vol. 286, no. 5448, 17 décembre 1999 (1999-12-17), pages 2331-2333, XP002191030 ISSN: 0036-8075
- WENGLER G ET AL: "AN ANALYSIS OF THE ANTIBODY RESPONSE AGAINST WEST NILE VIRUS E PROTEIN PURIFIED BY SDS-PAGE INDICATES THAT THIS PROTEIN DOES NOT CONTAIN SEQUENTIAL EPITOPES FOR EFFICIENT INDUCTION OF NEUTRALIZINGANTIBODIES" JOURNAL OF GENERAL VIROLOGY, READING, BERKS, GB, vol. 70, no. 4, 1989, pages 987-992, XP001050460 ISSN: 0022-1317
- DATABASE WPI Section Ch, Week 198505 Derwent Publications Ltd., London, GB; Class B04, AN 1985-030641 XP002191031 & SU 1 102 605 A (NIKOLAEV V P), 15 juillet 1984 (1984-07-15)

## Description

La présente invention est relative à une souche neuroinvasive et neurovirulente du virus West-Nile, dénommée IS-98-ST1, à des molécules d'acide nucléique issues de son génome, aux protéines et peptides codés par lesdites molécules d'acide nucléique ainsi qu'à leurs applications.

La présente invention concerne également tous variants de la souche virale IS-98-ST1 ayant au moins une mutation dans la séquence nucléique correspondant à NS5.

La famille des *Flaviviridae* regroupe les virus du genre flavivirus responsables de pathologies humaines graves telles que la dengue, la fièvre jaune, les encéphalites transmises par les tiques, l'encéphalite japonaise, l'encéphalite à West-Nile et les virus des hépatites C et G. Si les flavivirus sont susceptibles de provoquer une morbidité et une mortalité importantes chez l'homme, l'infection est généralement asymptomatique et seule une fraction des individus infectés développent une maladie grave.

Les flavivirus sont des petits virus enveloppés. Leur génome est une molécule d'ARN monocaténaire de polarité positive d'environ 11 000 bases. L'ARN génomique est associé à plusieurs copies de la protéine de capside C pour former la nucléocapside; elle est entourée d'une enveloppe virale constituée d'une double couche lipidique issue des membranes du réticulum endoplasmique (RE) dans lesquelles sont ancrées la protéine d'enveloppe E et la protéine de membrane M. L'ARN génomique des flavivirus contient un unique cadre de lecture ouvert d'environ 10500 nucléotides flanqué de deux courtes régions non codantes à ses extrémités 5' et 3'. Le génome est traduit en une polyprotéine d'environ 3400 acides aminés qui est le précurseur des protéines structurales C , prM (le précurseur intracellulaire de M) et E dans sa partie N-terminale et d'au moins sept protéines non structurales (NS) de NS1 à NS5 dans sa partie C-terminale.

Jusque très récemment, le virus West-Nile était reconnu comme un virus peu pathogène, responsable d'un syndrome grippal et présent en Afrique, en Europe du Sud et au Moyen Orient ; il a été isolé au cours d'épidémies, survenues notamment en Israël dans les années 1950 et en Afrique du Sud dans les années 1970.

Très récemment, l'épidémiologie du virus West-Nile s'est modifiée et un nombre croissant de cas d'encéphalites a été observé aux cours des épidémies survenues en Roumanie en 1996, en Israël en 1998 et aux USA en 1999.

Des souches pathogènes ont été isolées lors de ces épidémies (Anderson et al., et Lanciotti et al., Science, 1999, 286 : 2331-2333, 2333-2337), en particulier la souche NY1999 (GenBank n°AF202541, Lanciotti et al., précité), dont la pathogénicité serait corrélée à la présence d'un site de glycosylation NTS dans la protéine d'enveloppe E (Jordan et al., Viral Immunol., 2000, 13, 4 : 435-446).

Des facteurs viraux mal identifiés pourraient être responsables de la gravité de l'infection, alors que la constitution génétique de l'hôte (humain ou non-humain) contribuerait à la résistance à l'infection.

Toutefois, les données relatives à ces souches pathogènes récemment isolées n'ont pas permis de déterminer tous les facteurs viraux et les gènes de l'hôte impliqués dans la sensibilité/résistance à l'infection par les *Flaviviridae*.

Des modèles murins ont permis d'établir l'existence d'une résistance génétique à l'infection par les flavivirus. Il a été montré que certaines lignées de souris récemment dérivées de l'état sauvage et appartenant aux espèces *Mus musculus musculus* ou *Mus spretus* (Det, BSVR, BRVR, PRI, CASA/Rk et CAST/Ei) sont résistantes à l'infection par les flavivirus, alors que les lignées consanguines de laboratoire les plus courantes qui dérivent majoritairement de l'espèce *Mus musculus domesticus*, n'y résistent pas (Sangster et al., J.Virol., 1993,67 : 340-347).

La résistance est contrôlée par au moins un locus autosomal dénommé *Flv*, localisé sur le chromosome 5, chez la souris et trois allèles *Flv^{s}*, *Flv^{r}* et *Flv^{mr}* confèrent respectivement la sensibilité, la résistance et la résistance intermédiaire à l'infection par les flavivirus. En utilisant une souche du flavivirus de l'encéphalite de la Vallée de Murray et des souris issues du croisement en retour de la lignée de souris résistante C3H/RV avec les lignées de souris sensibles C3/He ou BALB/c, le locus *Flv* a été localisé dans une région de 0,9 cM du chromosome 5, chez la souris, entre les marqueurs *D5Mit68* et *D5Mit242* (G.R. Shellam et al., Rev. Sci. Tech. Off. Epiz. 1998,17 :231-248.).

Les Inventeurs ont maintenant isolé une nouvelle souche du virus West-Nile, à partir d'échantillons prélevés sur des cigognes en Israël (dans la ville d'Eilat) en septembre 1998, qui a été sélectionnée pour l'étude de la résistance/sensibilité d'un hôte (mammifère humain ou non-humain) à l'infection par les virus de la famille des *Flaviviridae*.

Conformément à l'invention, ladite souche neurovirulente et neuroinvasive du virus West-Nile isolée, dénommée IS-98-ST1, est caractérisée en ce que son génome est constitué par la séquence SEQ ID NO :1 qui code pour une polyprotéine présentant la séquence SEQ ID NO : 2.

Les Inventeurs ont notamment montré que les souris de laboratoire sont extrêmement sensibles à l'infection par la souche IS-98-ST1 alors que les souris des lignées SEG, WMP, STF et MAI qui dérivent de souris sauvages appartenant à des espèces différentes bien que du même genre *Mus*, sont complètement résistantes à l'infection par cette souche ; une inoculation par voie intrapéritonéale de 1000 UFF (Unités Formant Foyer ; UFF:DL50 = 100) est mortelle à 100 % pour les souris de laboratoire, alors que les souris sauvages ne présentent aucun symptôme ; en outre, le virus se réplique chez ces souris, comme le montre l'apparition d'anticorps sériques spécifiques.

La présente invention a également pour objet des réactifs, dérivés de la souche IS-98-ST1, utilisés pour l'étude et le diagnostic des infections par les *Flaviviridae*, lesquels réactifs sont sélectionnés dans le groupe constitué par les réactifs suivants :
(a) une molécule d'acide nucléique choisie parmi la séquence SEQ ID NO :1, les fragments d'au moins 15 nucléotides de la séquence SEQ ID NO :1 et les séquences complémentaires sens et anti-sens des séquences précédentes, à l'exclusion du fragment présentant la séquence GENBANK AF205882.
(b) un vecteur recombinant comprenant une molécule d'acide nucléique telle que définie en (a),
(c) une cellule transformée par une molécule d'acide nucléique telle que définie en (a), un vecteur tel que défini en (b) ou une souche neurovirulente du virus West-Nile telle que définie en (a),
(d) une protéine ou un peptide codé par une molécule d'acide nucléique telle que définie en (a),
(e) un anticorps polyclonal, susceptible d'être obtenu par immunisation d'un mammifère non-humain avec la souche IS-98-ST1 du virus West-Nile telle que définie ci-dessus ; de manière préférée, ledit mammifère non-humain est une souris homozygote pour l'allèle *Flv^{r}*, résistante à l'infection par les *Flaviviridae*, et
(f) un anticorps polyclonal ou monoclonal, susceptible d'être obtenu par immunisation d'un mammifère non-humain avec un vecteur recombinant tel que défini en (b) ou bien une protéine ou un peptide, tels que définis en (d).

Ces différents réactifs sont préparés et utilisés selon les techniques classiques de biologie moléculaire et d'immunologie, en suivant les protocoles standards tels que ceux décrits dans Current Prolocols in Molecular Biology (Frederick M. AUSUBEL, 2000, Wiley and son Inc, Library of Congress, USA) et dans Current Protocols in Immunology (John E. Coligan, 2000, Wiley and Son Inc. Library of Congress. USA).

Les fragments d'acides nucléiques tels que définis ci-dessus, en particulier ceux correspondant aux séquences SEQ ID NO: 3-11 sont utilisés par exemple comme sonde ou comme amorce pour le diagnostic d'une infection par le virus West-Nile ; l'infection est détectée par exemple par PCR et/ou par hybridation, à partir des acides nucléiques extraits d'un échantillon biologique prélevé chez un individu susceptible d'être infecté ou un animal de laboratoire inoculé par ledit virus.

Selon un mode de réalisation avantageux desdits fragments, ils comprennent au moins 15 nucléotides de la SEQ ID NO:1 situés entre 10 et 100 nucléotides en amont ou en aval de l'un des codons en position suivante :
- codon Asparagine (N) en positions 2518-2520, correspondant au résidu en position 17 de la protéine NS1 ou en position 808 de la séquence SEQ ID NO:2 (la séquence de la protéine NS1 s'étend des codons en position 792 à 1144 de la séquence SEQ ID NO:2, correspondants aux nucléotides 2470 à 3528 de la séquence SEQ ID NO:1).
- codon Arginine (R) en positions 4018-4020, correspondant au résidu en position 164 de la protéine NS2A ou en position 1308 de la séquence SEQ ID NO:2 (la séquence de la protéine NS2A s'étend des codons en position 1145 à 1374 de la séquence SEQ ID NO:2, correspondants aux nucléotides 3529 à 4218 de la séquence SEQ ID NO:1),
- codons Glycine (G) en positions 4462-4464 et acide Glutamique (E) en positions 4465-4467, correspondant respectivement au résidu en position 82 et 83 de la protéine NS2B ou en position 1456 et 1457 de la séquence SEQ ID NO:2 (la séquence de la protéine NS2B s'étend des codons en position 1375 à 1505 de la séquence SEQ ID NO:2, correspondants aux nucléotides 4219 à 4611 de la séquence SEQ ID NO:1),
- codons Proline (P) en positions 6097-6099 et acide Glutamique en positions 6172-6174, correspondant respectivement au résidu en position 496 et 521 de la protéine NS3 ou en position 2001 et 2026 de la séquence SEQ ID NO:2 (la séquence de la protéine NS3 s'étend des codons en position 1506 à 2124 de la séquence SEQ ID NO:2, correspondants aux nucléotides 4612 à 6468 de la séquence SEQ ID NO:1), et
- codons Serine (S) en positions 7840-7842, Asparagine (N) en positions 8518-8520 et Alanine (A) en positions 8794-8796, correspondant respectivement au résidu en position 54, 280 et 372 de NS5 ou en position 2582, 2808 et 2900 de la séquence SEQ ID NO:2 (la séquence de la protéine NS5 s'étend des codons en position 2529 à 3430 de la séquence SEQ ID NO:2, correspondants aux nucléotides 7681 à 10386 de la séquence SEQ ID NO:1).

De telles amorces sont utiles pour amplifier des fragments contenant lesdits codons.

Lesdites amorces sont situés entre 10 et 100 nucléotides en amont ou en aval desdits codons.

Selon un autre mode de réalisation avantageux desdits fragments, ils sont constitués par les fragments comprenant les codons précités, de préférence entre 50 et 200 nucléotides, qui sont amplifiés en utilisant les amorces telles que définies ci-dessus.

Les inventeurs decrivent également des Fragments comprenant au moins 15 nucléotides de la SEQ ID No. 1 en amont ou en aval du codon Alanine (A) en positions 1117-1119, correspondant au résidu en position 51 de la protéine E ou en position 341 de la séquence de la polyprotéine virale de séquence SEQ ID NO:2 (la séquence de la protéine E s'étend des codons en position 291 à 791 de la séquence SEQ ID NO:2, correspondants aux nucléotides 967 à 2469 de la séquence SEQ ID NO:1).

Les vecteurs recombinants tels que définis ci-dessus, en particulier les vecteurs d'expression et les cellules transformées par lesdits vecteurs d'expression sont avantageusement utilisés pour la production des protéines et des peptides correspondants.

Lesdites protéines et lesdits peptides, qui sont aptes à être reconnus et/ou à induire la production d'anticorps spécifiques du virus West-Nile, en particulier de souches neurovirulentes, sont utiles pour le diagnostic d'une infection par un virus West-Nile; l'infection est détectée par une technique appropriée, notamment EIA, ELISA, RIA, immunofluorescence à partir d'un échantillon biologique prélevé chez un individu susceptible d'être infecté ou un animal de laboratoire inoculé par ledit virus. Les protéines et les peptides tels que définis ci-dessus sont également utilisés pour la recherche de partenaires cellulaires de ces protéines virales susceptibles d'être impliqués dans la pathogénicité (neurovirulence) du virus West-Nile; ces partenaires sont identifiés par des techniques d'immunoaffinité, par exemple par chromatographie sur colonne d'immunoaffinité.

Les anticorps selon l'invention sont utiles pour le diagnostic d'une infection par un virus West-Nile en particulier des souches neurovirulentes; l'infection est détectée par une technique appropriée, notamment EIA, ELISA, RIA, immunofluorescence à partir d'un échantillon biologique prélevé chez un individu susceptible d'être infecté ou un animal de laboratoire inoculé par ledit virus. Parmi ceux-ci, les anticorps produits par immunisation de souris *Flv^{r}*/*Flv^{r}* avec la souche IS-98-ST1 possèdent avantageusement un titre élevé et une très grande spécificité pour le virus West-Nile.

Les cellules transformées selon l'invention, en particulier les cellules neurales (neurones et cellules endothéliales) infectées par une souche neurovirulente telle que définie ci-dessus, sont utilisées pour identifier les gènes issus de ces cellules dont l'expression pourrait être modulée au cours de l'infection virale ; ces gènes sont détectés par exemple par la technologie des biopuces, selon les protocoles classiques tels que décrits dans « Atlas Mouse Arrays (#membranes) ATLAS^{™} NYLON cDNA EXPRESSION ARRAYS (CLONTECH, USA).

La présente invention a également pour objet un modèle d'étude non humain de la sensibilité/résistance à l'infection par un virus de la famille des *Flaviviridae*, **caractérisé en ce qu**'il comprend au moins une souche neurovirulente du virus West-Nile telle que définie ci-dessus.

Selon un mode de réalisation avantageux dudit modèle, il comprend en outre une souris homozygote pour l'allèle *Flv^{r}* ou *F*/*v^{s}*.

La présente invention a également pour objet un procédé de détection d'une infection à virus West-Nile, **caractérisé en ce qu**'il comprend :
- l'amplification des ARN issus d'un échantillon biologique à tester à l'aide des amorces telles que définies ci-dessus, et
- le séquençage du produit d'amplification obtenu.

Une telle détection peut avantageusement permettre le pronostic de la sévérité d'une encéphalite virale à virus West-Nile.

La souche neurovirulente du virus West-Nile selon l'invention est utilisée pour le criblage de gènes cellulaires impliqués dans la résistance d'un mammifère à l'infection par un virus de la famille des *Flaviviridae*, de préférence le virus de l'hépatite C.

De manière avantageuse, ledit procédé de criblage comprend les étapes suivantes :
- mise en culture de cellules dérivées d'un hôte (humain ou non-humain) sélectionné pour sa résistance ou sa sensibilité à l'infection par un *Flaviviridae*,
- infection *in vitro* desdites cellules par un *Flaviviridae*, et
- détection de gènes exprimés de manière différentielle dans lesdites cellules infectées.

Conformément à l'invention, ladite détection peut comprendre l'établissement du profil de transcrits ou de protéines à partir desdites cellules.

La présente invention a également pour objet l'utilisation du modèle tel que défini ci-dessus pour le tri de molécules actives contre une infection virale due à un virus de la famille des *Flaviviridae*.

La présente invention a en outre pour objet un procédé de tri de molécules actives contre une infection par un Flavivirus , caractérisé par :
- la mise en contact d'une culture de cellules eucaryotes, issues d'un mammifère (humain ou non-humain) sensible à l'infection à un *Flaviviridae* avec une suspension virale de la souche selon la revendication 1, en présence ou en l'absence de la molécule à tester et
- détection de l'amplification/réplication du virus, par toute méthode connue (quantification génome, ARNm,-protéines, particules virales).

La présente invention a également pour objet un variant de la souche virale telle que définie ci-dessus, **caractérisé en ce que** son génome comprend au moins une mutation dans la séquence nucléotidique correspondant à la protéine NS5.

Outre les dispositions qui précèdent, l'invention comprend encore d'autres dispositions, qui ressortiront de la description qui va suivre, qui se réfère à des exemples de mise en oeuvre de l'objet de la présente invention, ave références aux dessins annexés dans lesquels :
- les figures 1A à 1E représentent la comparaison de la séquence en acides aminés des protéines virales de la souche IS-98-ST1 (SEQ ID NO : 2), isolée à partir de cigognes (CI) et de la souche New York (NY99 ; Genbank AF196835) isolée à partir de flamands roses (FLA), lors de l'épidémie de 1999 aux Etats-Unis,
- la figure 2 représente la cinétique de mortalité et la cinétique d'apparition des anticorps sériques spécifiques chez des souris sensibles *Flv*^{s}/*Flv*^{s} (BALB/c), infectées par la souche IS-98-ST1 du virus West-Nile,
- la figure 3 représente la cinétique de propagation de la souche IS-98-ST1 dans le système nerveux central des souris sensibles *Flv*^{s}/*F*/*v*^{s} (BALB/c),
- les figures 4 (A. B et C) représentent la cinétique d'apparition des antigènes viraux dans les cellules Neuro 2a et les neurones primaires de souris sensibles (BALB/c) infectées par le virus West-Nile (souche IS-98-ST1),
- la figure 5 représente la mort par nécrose des cellules Neuro 2a infectées par le virus West-Nile (souche IS-98-ST1),
- la figure 6 représente le protocole expérimental utilisé pour préciser la localisation du locus *Flv* sur le chromosome 5 de la souris,
- la figure 7 représente la carte génétique du locus *Flv*, déterminée à partir de souris sensibles, issues du premier croisement en retour entre les lignées résistantes (MAI/Pas et MBT/Pas) et les lignées sensibles (C57BL/6 ou BALB/c). Les boîtes blanches représentent les allèles BALB/c ou C57B1/6 et les boîtes noires représentent les allèles MAI/Pas ou MBT/Pas.
- la figure 8 représente la carte génétique du locus *Flv* déterminée à partir des souris résistantes et sensibles, issues du premier croisement en retour (BC1) entre les lignées résistantes (MAI/Pas et MBT/Pas) et les lignées sensibles (C57BL/6 et BALB/c). Les lignes grisées représentent les allèles (BALB/c ou C57B1/6) et les lignes noires représentent les allèles MAI/Pas ou MBT/Pas,
- la figure 9 représente la carte génétique et la carte physique du locus *Flv* et la position du gène OAS dans ce locus, et
- la figure 10 représente la distribution des allèles *Flv* chez les souris résistantes et sensibles issues du premier croisement en retour (BC1) entre les lignées résistantes (MAI/Pas et MBT/Pas) et les lignées sensibles (C57BL/6 et BALB/c).

### Exemple 1 : Isolement, amplification, purification et titration de la souche neuroinvasive du virus West-Nile IS-98-ST1

Un isolat du virus West-Nile (WN) a été obtenu à partir du système nerveux central d'une cigogne manifestant des troubles neuropathologiques sévères, en septembre 1998, à Eilat (Israël). L'infection de cellules VERO par cet isolat est cytolytique et l'immunofluorescence indirecte avec un ascite de souris immun spécifique du virus West-Nile (sérum immun de référence WN 8907) est positive à 100 %. Le virus produit sur cellules VERO a été récolté et amplifié sur cellules de moustiques AP61 (Després et al., Virol., 1993, 196 : 209-219)

Le Passage 1 (ou P1) du virus WN sur cellules AP61 a été récolté 3 jours après l'infection ; il possède un titre de 2,5 x 10⁸ UFF/ml (Unité Formant Foyer) par la technique de titration sur cellules AP61 décrite dans Després et al (précité). L'inoculum P1 du virus WN sur cellules AP61 a été identifié comme la souche IS-98-ST1.

Un P2 a été obtenu à partir de cellules AP61 infectées par la souche IS-98-ST1. P1 (titre: 6 x 10⁷ UFF/ml). L'inoculum P2 de IS-98-ST1 est utilisé pour les épreuves de sensibilité à l'infection virale chez des souris adultes.

Un inoculum viral P3 de la souche IS-98-ST1 avec un titre de 5 x 10⁷ UFF/ml a été produit sur cellules AP61. Une préparation virale hautement purifiée, préparée selon le protocole de purification des flavivirions décrit dans Després et al., 1993) a été obtenue à partir de 20 boites de 150 cm² de cellules AP61 récoltées 3 jours après l'infection par l'inoculum P3 du virus WN souche IS-98-ST1 (multiplicité d'infection de 0,4). La souche IS-98-ST1 purifiée en gradients de saccharose a un titre final de 2 x 10¹⁰ UFF/ml. Les ARN extraits de ce virus purifié sont utilisés pour amplifier les ADNc correspondant aux protéines virales C, prM et NS1 ou aux séquences non codantes aux extrémités 5' et 3' du génome viral.

### Exemple 2 : Séquençage du génome de la souche neuroinvasive IS-98-ST1

Le génome viral a été extrait à partir du surnageant de culture des cellules VERO infectées de l'exemple 1 à l'aide du kit "QIAamp Viral RNA" (QIAGEN), en suivant les instructions du fabricant. 6 produits RT-PCR chevauchants ont été amplifiés à partir de ces ARNs en utilisant les amorces décrites par Lanciotti et al. (précité). Les extrémités 5' et 3' du génome viral ont été amplifiées respectivement à l'aide des amorces suivantes:
- 5' AGTAGTTCGCCTGTGTGAGCTGACAAAC 3' (SEQ ID NO:5), et
- 5' AGATCCTGTGTTCTCGCACCACCAGCCAC 3' (SEQ ID NO:6).

L'ADNc correspondant à la protéine virale E a été amplifié à l'aide des amorces 5' GGATGGATGCT(A/T)GG(G/T)AGCAAC 3' (SEQ ID NO:7) et 5' CCATCCAAGCCTCCACATC 3' (SEQ ID NO:8), s'hybridant respectivement dans le gène de la protéine M (positions 889 à 909 de la séquence SEQ ID NO:1) et dans le gène de la protéine NS1 (positions 2539 à 2557 de la séquence SEQ ID NO:1).

Les ADNc obtenus ont été purifiés par chromatographie échangeuse d'ions et précipités dans 2 volumes d'isopropanol. Ensuite les ADNc ont été séquencés sur les deux brins en utilisant le kit "Taq Dye Deoxy Terminator Cycle Sequencing " (PERKIN ELMER CORP./APPLIED BIOSYSTEM) et les amorces espacées de 400 paires de bases sur le génome viral (Lanciotti et al., précité). Le séquençage a été réalisé avec 0.2. pmoles d'ADNc purifié et 30 pmoles d'amorces, en suivant le protocole recommandé par le fabricant. L'alignement des séquences est réalisé à l'aide du logiciel CLUSTAL W.

La séquence génomique complète de la souche IS-98-ST1 du virus West-Nile correspond à la séquence SEQ ID NO :1.

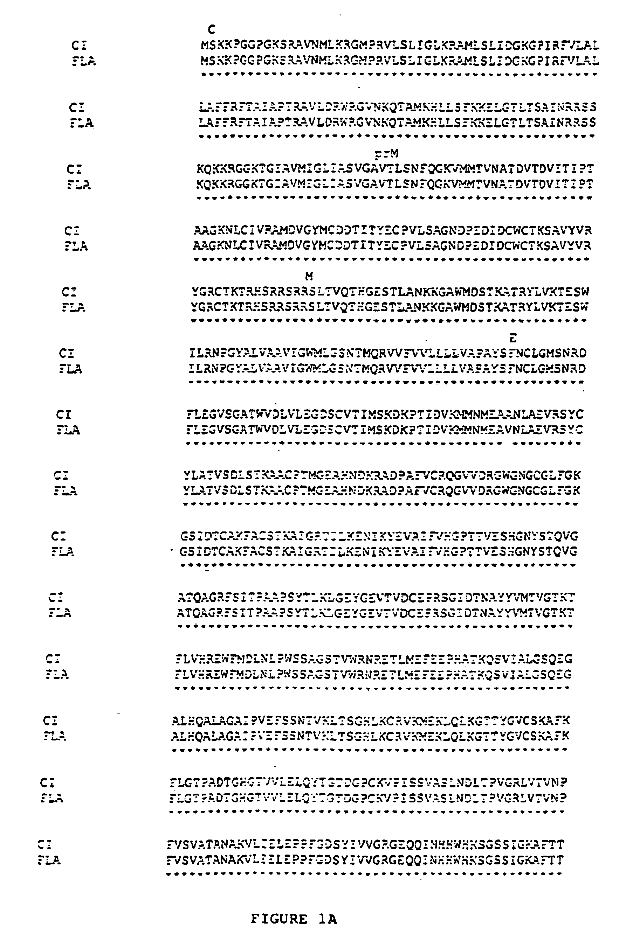

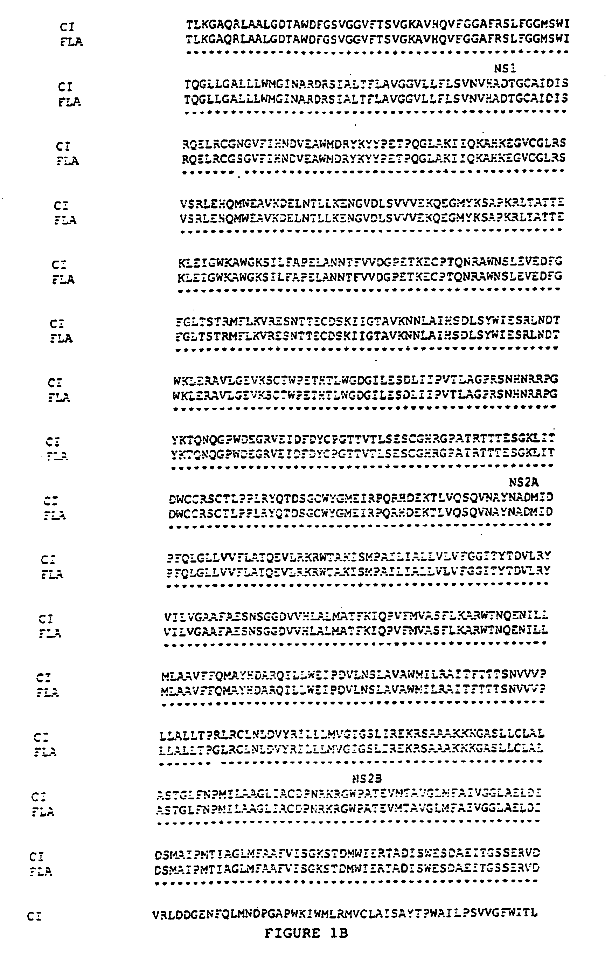

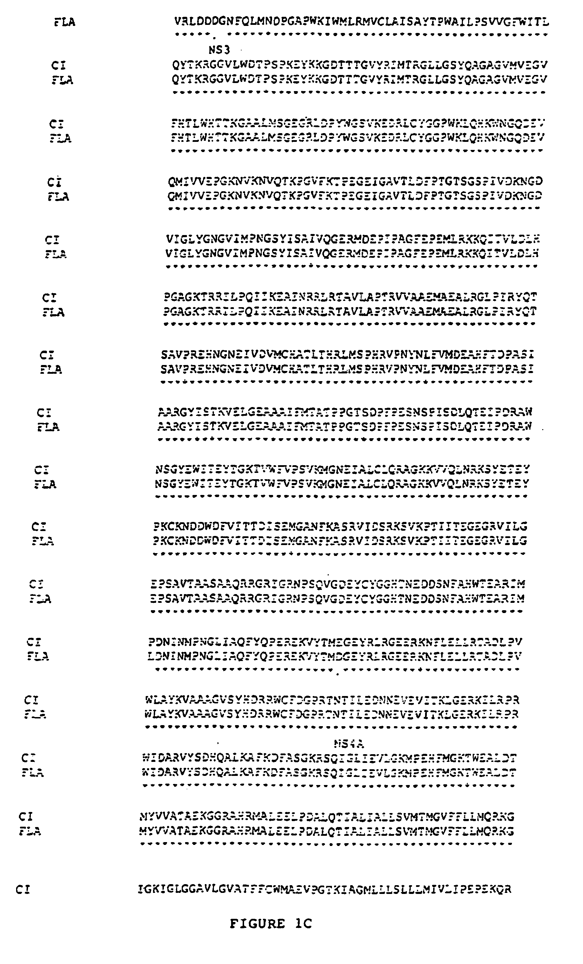

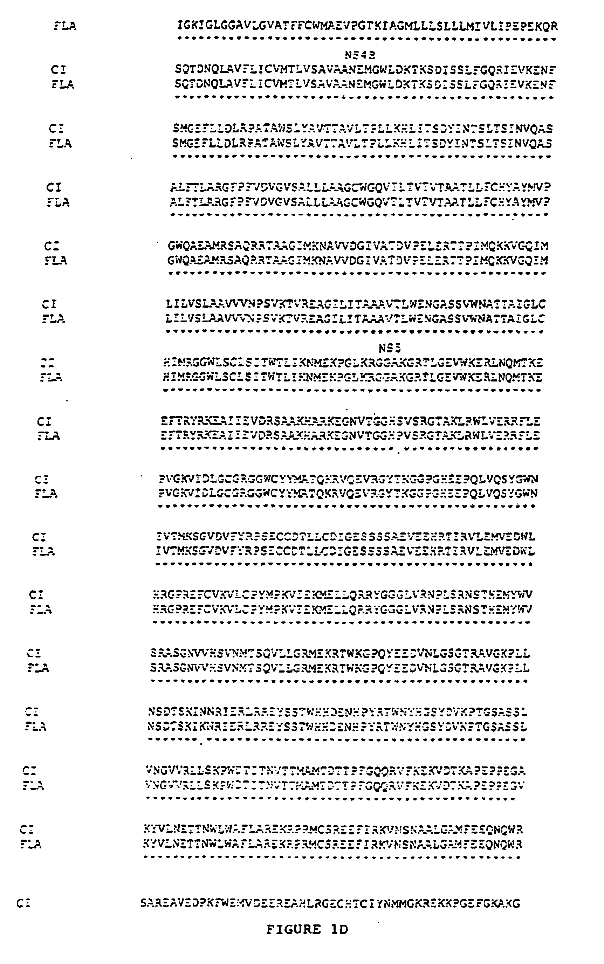

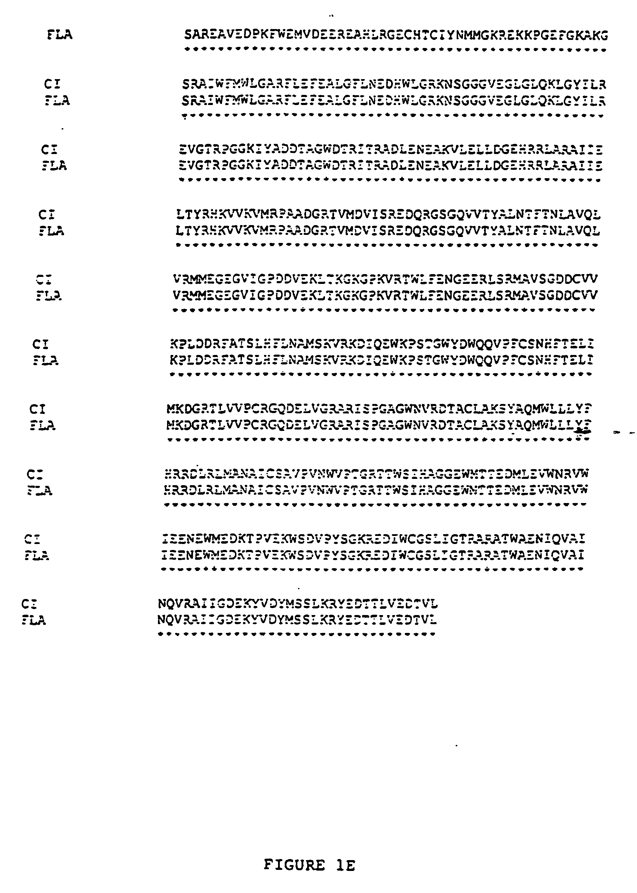

L'alignement des séquences en acides aminés de la souche IS-98-ST1 (Seq ID NO : 2) et de la souche NY99, présentée à la figure 1, montre que la souche IS-98-ST1 isolée en Israël en 1998 et la souche NY-99 isolée à New York en 1999 sont très proches (divergence de moins de 0,2% au niveau des séquences en acides aminés).

Cependant, les différences observées dans la souche IS-98-ST1, respectivement dans les protéines E (A₅₁), NS1 (N₁₇), NS2A (R₁₆₄), NS2B (G₈₂, E₈₃), NS3 (P₄₉₆, E₅₂₁) et NS5 (S₅₄, N₂₈₀, A₃₇₂) sont potentiellement responsables de la neurovirulence et des propriétés neuroinvasives observées avec cette souche et peuvent servir de marqueur de virulence du virus West-Nile.

### Exemple 3 : Clonage des protéines de la souche neuroinvasive IS-98-ST1 et utilisations des plasmides recombinants obtenus.

### 1- La protéine C

L'ARN génomique extrait des virions IS-98-ST1 purifiés sur gradients de saccharose décrits à l'exemple 1, à l'aide de la solution RNA PLUS 2 (Q.BIOGEN), est utilisé comme matrice pour amplifier la séquence codant pour la protéine C (acides aminés 1 à 123) par la technique RT-PCR (kit Titan One Tube RT-PCR; Roche Biochemicals #1939 823).

Le couple d'amorces utilisé sur la matrice ARN est le suivant :
- 5'C/WNV (séquence des nt 81-117 de la séquence SEQ ID NO:1)
   5' TAG CAC GAA GAA TTC GAT GTC TAA AAA CCA GGA GGG 3' (SEQ ID NO:11)
   qui contient le site de restriction *EcoR*I, et
- 3'C/WNV (séquence anti-sens des nt 433 à 482 de la séquence SEQ ID NO:1)
   5' AAGTTAGCCCGGGTTAATGCTCCTACGCTGGCGATCAGGCCAATCAGGAC 3' (SEQ ID NO:4) qui contient le site de restriction *Sma*I.

L'ADNc de la protéine C de la souche IS-98-ST1 (acides aminés 1 à 123) du virus WN a été cloné d'une part entre les sites *EcoR*I et *Sma*I du plasmide pCI-neo (Promega # E1841) et d'autre part entre les sites *Ksp*I et *Sma*I du plasmide pIVEX 2.4a (Roche).

Le plasmide recombiné pCI-C/WN (déposé le 21 juin 2001 à la Collection Nationale de Culture de Microorganismes de l'Institut Pasteur de Paris, 28, rue du Docteur Roux, 75724, PARIS Cedex 15 sous le numéro 1-2688) contient la séquence complète du gène de la protéine C de la souche IS-98-ST1 du virus WN entre les promoteurs T7 et T3. La transcription *in vitro* de pCI-C/WN linéarisé par *Nhe*I sous la dépendance du promoteur T3 synthétise un ARN d'environ 350 bases complémentaires de la séquence virale génomique. La ribosonde marquée à la DIG (digoxigénine) est utilisée pour la détection des ARN viraux sens positif présents dans les cellules infectées par le virus WN, par la technique d'hybridation *in situ*, selon le protocole décrit dans Després et al., (J. Virol., 1998, 72 : 823-829).

Le plasmide recombinant pIVEX-C/WN est utilisé pour la production massive de la protéine C (acides aminés 1 à 123) du virus WN en lysat bactérien (système RTS 500 de Roche). La protéine recombinante C produite *in vitro* possède à son extrémité N-terminale une séquence [His]₆ et le site de clivage reconnu par la protéase Xa pour permettre d'une part sa purification sur colonne de Ni et d'autre part l'élimination des résidus histidines. La protéine C de la souche IS-98-ST1 du virus WN ainsi produite est utilisée pour des études structurales, pour la recherche de partenaires cellulaires de cette protéine en colonne d'immunoaffinité, et pour la production d'anticorps monospécifiques chez le lapin.

### 2- La protéine M

Les ADNc de la souche IS-98-ST1 du virus WN codant pour la protéine M (acides aminés 215 à 290 de la polyprotéine virale) ou son ectodomaine de 41 acides aminés (acides aminés 215 à 255; acronyme *ectoM*) sont clonés :
(1) en phase avec l'extrémité C-terminale de l'EGFP dans le plasmide p[95-114]EGFP, dérivé du plasmide pEGFP-N1 (Clontech) qui comprend les résidus 95-114 de la protéine C du virus de la dengue de type 1 (souche BR/90) fusionnés en phase avec la séquence N-terminale de la protéine EGFP[215-290]WNV, pour donner le plasmide p[95-114]EGFP[215-290]WNV,
(2) dans le plasmide pIVEX (système RTS 500 de Roche) pour donner le plasmide pIVEX[EGFP][215-255]WNV,
(3) dans le vecteur rétroviral TRIPdeltaU3CMV, pour donner le plasmide TRIPdeltaU3CMV[95-114]EGFP[215-255]WNV.

Le plasmide pIVEX[EGFP][215-255]WNV permet la synthèse *acellulo* et la purification de la protéine chimérique EGFP-*ectoM* WNV qui est utilisée d'une part pour la production d'anticorps monospécifiques dirigés contre la protéine M du virus WN et d'autre part pour la recherche de partenaires cellulaires de la molécule *ectoM* WNV en colonne d'immunoaffinité.

Le plasmide TRIPdeltaU3CMV[95-114]EGFP[215-255]WNV est cotransfecté dans des cellules 293T avec les plasmides 8.7 et G-VSV pour produire des particules virales pseudotypées par l'enveloppe G du virus de stomatite vésiculaire (VSV), contenant les protéines internes du virus de l'immunodéficience acquise (VIH) et des molécules d'ARN chimériques CMV[95-114]EGFP[215-255]WNV. L'infection des cellules cibles par le vecteur recombiné non réplicatif permet l'intégration dans le génome cellulaire de l'ADN CMV[95-114]EGFP[215-255]WNV et l'expression stable de l'ectodomaine de la protéine M-WN sous le contrôle du promoteur CMV.

### 3- La protéine NS 1

L'ARN génomique extrait des virions IS-98-ST1 purifiés est amplifié par la technique RT-PCR (kit Titan One Tube RT-PCR; Roche Biochemicals #1939 823) à l'aide du couple d'amorces suivant:
- 5'TGGATGGGATCCAATATGCGTGATAGGTCC 3' (SEQ ID NO:9) qui contient le site de restriction *BamHl* et
- 3'AAAAGGGTCAATGGTACCAGCATTTTAAGCATTCACGTT 3' (SEQ ID NO:10) qui contient le site de restriction *KpnI*.

L'ADNc codant pour la glycoprotéine NS1 avec son peptide signal (acides aminés 767 à 1143 de la polyprotéine virale est cloné entre les sites *BamH*1 et *Kpn*I du vecteur rétroviral TRIPdeltaU3 pour produire le plasmide recombiné TRIPdeltaU3-CMV-NS1-WN (déposé le 9 janvier 2002 à la Collection Nationale de Culture de Microorganismes de l'Institut Pasteur de Paris, 28 rue du Docteur ROUX, 75724, PARIS Cedex 15 sous le numéro I-2770). Le plasmide TRIPdeltaU3-CMV-NS1-WN est cotransfecté dans des cellules 293T avec les plasmides 8.7 et G-VSV pour produire des particules virales pseudotypées par l'enveloppe G du virus de stomatite vésiculaire (VSV), contenant les protéines internes du virus de l'immunodéficience acquise (VIH) et des molécules d'ARN chimériques CMV-NS1-WN. L'infection des cellules cibles par le vecteur recombiné non réplicatif permet l'intégration dans le génome cellulaire de l'ADN CMV-NS1-WN et l'expression stable de la protéine NS1 du virus WN sous le contrôle du promoteur CMV. La protéine NS1 de la souche IS-98-ST1 du virus WN ainsi produite est utilisée pour des études structurales, pour la recherche de partenaires cellulaires de cette protéine en colonne d'immunoaffinité, et pour la production d'anticorps monospécifiques chez le lapin.

### Exemple 4 : Les souris sauvages et de lignées consanguines de laboratoire se différencient par leur sensibilité à l'infection par la souche neuroinvasive IS-98-ST1 du virus West-Nile.

### 1- Les lignées de souris et les cellules sensibles.

### a) lignées de souris sensibles

Des souris de lignées consanguines sensibles *Flv*^{s} (BALB/c) âgées de 6 semaines sont inoculées par la voie intrapéritonéale avec 100 UFF de la souche IS-98-ST1 virus West-Nile (UFF:DL50 = 10), préparée comme décrit à l'exemple 1.

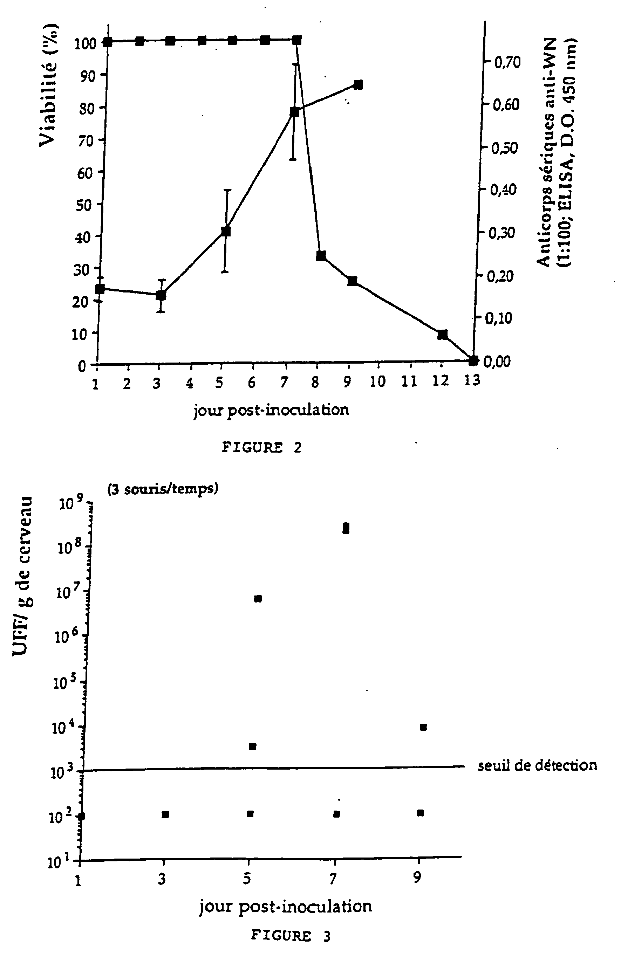

Ces souris meurent à 100% avec un temps moyen de mortalité de 9 ± 2 jours (Figure 2).

La cinétique de propagation de la souche IS-98ST1 dans le système nerveux central de la souris sensible (BALB/c) a été analysée à partir des extraits de cerveau des souris infectés titrés sur cellules AP61, selon la technique décrite dans selon la technique décrite dans Després et al. (J. Virol., 1998, 72, 823-829). Les résultats montrent que le virus est détecté dans le système nerveux central (SNC) murin au 5^{ème} jour de l'infection et la production virale est maximale au 7^{ème} jour (Figure 3). Au 9^{ème} jour de l'infection, le virus n'est plus détecté dans le SNC murin (Figure 3).

La réplication du virus WN dans le SNC et les organes périphériques des souris infectées par la souche IS-98-ST1 est également détectée par immuno-histologie, selon les protocoles classiques tels que décrits dans Després et al., 1998 (précité) et par hybridation *in situ*, selon les protocoles décrits à l'exemple 3.

Les anticorps sériques spécifiquement dirigés contre les protéines du virus WN sont titrés par ELISA selon le protocole décrit dans Després et al., 1993 (précité), en utilisant la souche IS-98-ST1 purifié sur gradient de saccharose telle que décrite à l'exemple 1. comme antigène. Les résultats montrent que les anticorps sériques apparaissent au 5^{ème} jour de l'infection et sont significativement détectés au 7^{ème} jour (figure 2).

### b) cellules sensibles

### b1) cultures primaires

Des neurones primaires et des astrocytes du SNC de souris sensibles homozygotes pour l'allèle *Flv^{s}* (souris Swiss, Janvier) sont préparés selon les protocoles classiques. Les cellules sont infectées par la souche IS-98-ST1 à une multiplicité d'infection de 20 UFF par cellule (m.i. de 20). L'effet cytopathique est observé en microscopie optique, la production virale est analysée par titration sur cellules AP61 comme décrit précédemment à l'exemple 1 et l'expression des antigènes viraux est analysée par radioimmunoprécipitation à l'aide d'un sérum immun de souris anti-West-Nile, selon les protocoles classiques tels que décrits dans Duarte Dos Santos et al. (Virology, 2000. 274 : 292-308).

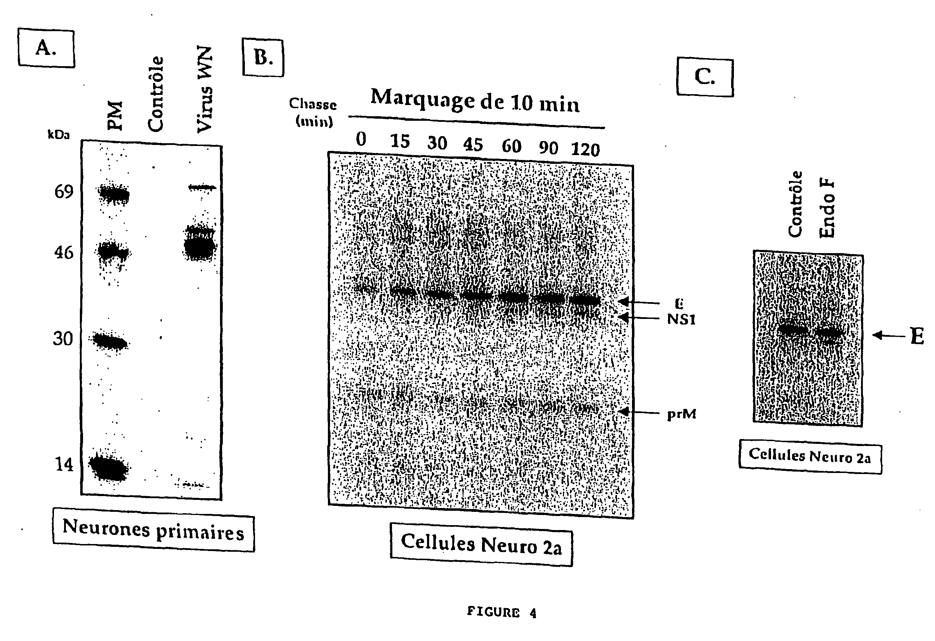

Les résultats montrent que 80% des neurones en culture produisent les antigènes viraux :
- leur profil en gel de polyacrylamide-SDS est présenté à la figure 4A.
- la production virale est de [3.0 ± 1,5] x 10⁶ UFF/ml après 20 h d'infection et de [7,0 ± 0.5] x 10⁷ UFF/ml à 40 h.
- les effets cytopathiques (ECPs) de type nécrotique sont observés après 48 h d'infection virale.

En revanche, les astrocytes du SNC murin ne sont pas permissifs à la réplication du virus WN souche IS-98-ST1.

### b₂) lignées cellulaires

Des cellules de neuroblastome murin Neuro 2a et des cellules d'hépatome humain HepG2, cultivées dans les conditions classiques telles que décrites dans Marianneau et al. (J. Virol., 1996, 77 : 2547-2554) sont infectées à différentes multiplicité d'infection par le virus WN souche IS-98-ST1, préparé comme décrit à l'exemple 1. L'effet cytopathique est observé en microscopie optique, la production virale est analysée par titration sur cellules AP61 comme décrit précédemment à l'exemple 1 et l'expression des antigènes viraux est analysée par radioimmunoprécipitation à l'aide d'un sérum immun de souris anti-West-Nile, selon les protocoles classiques tels que décrits dans Duarte Dos Santos et al., Virol., 2000, 274,292-308.

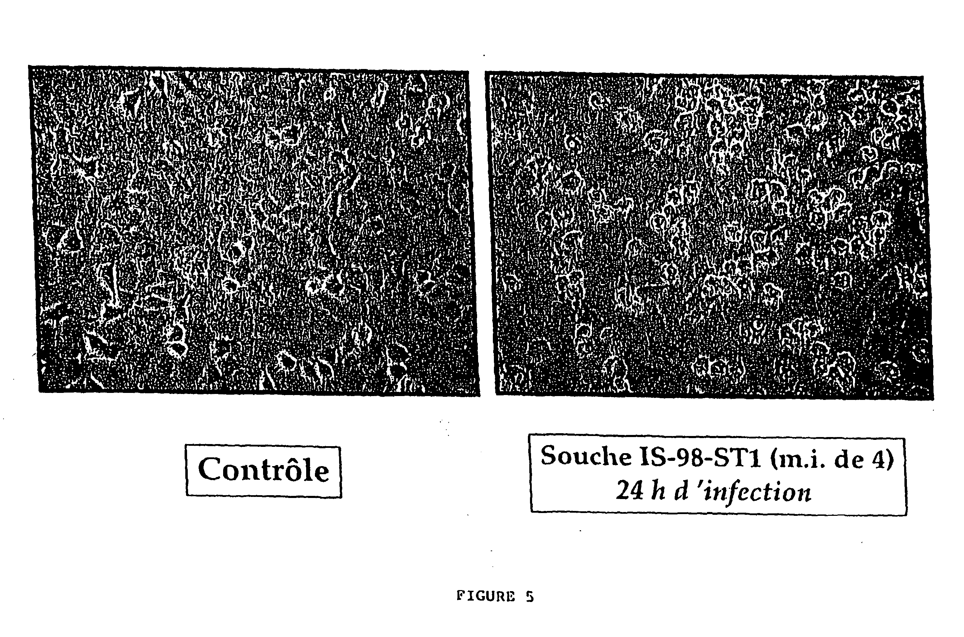

Les résultats montrent que les cellules de neuroblastome murin Neuro 2a sont permissives à la réplication de la souche IS-98-ST1 du virus WN. Une m.i. de 4 est nécessaire pour infecter 80% des cellules Neuro 2a en monocouche. La production virale est de 10⁷ UFF/ml (m.i. de 4) après 40 h d'infection et la mort cellulaire par nécrose est massive (Figure 5). La cinétique de production des antigènes majeurs prM, E et NS1 à partir de la polyprotéine virale présentée dans la figure 4B. montre que le demi-temps de formation de la glycoprotéine d'enveloppe E est d'environ 30 min. La protéine E de la souche IS-98-ST1 semble ne posséder qu'un seul résidu N-glycanne (figure 4C).

Les résultats montrent également que les cellules d'hépatome humain HepG2 sont permissives à la réplication de la souche IS-98-ST1 du virus M/N. A une m.i. de 10, la production virale est de [2 ± 1] x 10⁶ UFF/ml après 48 h d'infection et les ECPs sont observés à partir de 72 h.

### 2- Les souris résistantes.

Les lignées de souris résistantes (*Flv^{r}*) qui dérivent de souris sauvages de l'espèce *Mus spretus* (SEG/Pas et STF/Pas), *Mus musculus musculus* (MBT/Pas, MAI/pas). *Mus musculus domesticus* (WMP/Pas), sont inoculées par la voie intrapéritonéale, avec 1000 UFF (100 DL50) de la souche IS-98-ST1 préparée selon le protocole décrit à l'exemple 1.

Contrairement aux souris de laboratoire qui sont sensibles à l'infection par la souche IS-98-ST1 et meurent en une dizaine de jours, ces souris dérivant de souris sauvages sont résistantes à l'inoculation de la souche IS-98-ST1 et néanmoins permissives à la réplication de la souche IS-98-ST1. En effet, l'infection virale des souris dérivant de souris sauvages est asymptomatique bien que le virus se multiplie *in toto* comme le démontre la production d'anticorps sériques anti-WN à hauts titres ; en ELISA, les titres des sérums à la dilution 1:100 pour 10⁶ UFF de virion purifié IS-98-ST1 sont supérieurs à 1 unité de D.O. à 450 nm.

Les souris résistantes à l'infection virale sont utilisées pour la production de sérums immuns spécifiquement dirigés contre les protéines de la souche IS-98-ST1 du virus WN. Trois semaines après inoculation du virus WN, les sérums prélevés de souris résistantes (0,045 ml par souris) sont mélangés, décomplémentés 30 min à 56°C puis dilués au 1:10 dans du DPBS* (^{V}/_{V}) supplémenté avec 0,2% (^{V}/_{V}) de Sérum Albumine bovine (Life Technologies) et 0,05% (^{P}/_{V}) d'azide de sodium. Les sérums dilués sont répartis en 0,2 ml et conservés à -20°C. Les sérums immuns dirigés contre la souche IS-98-ST1 sont utilisés aux dilutions finales de 1:500 pour l'immunofluorescence indirecte et au 1:1000 pour l'immunoprécipitation des protéines virales radiomarquées.

### Exemple 5 : Utilisation de la souche IS-98-ST1 du virus West-Nile pour identifier les gènes cellulaires impliqués dans la sensibilité de l'hôte à l'infection aux virus de la famille des Flaviviridae.

### 1) Méthodes

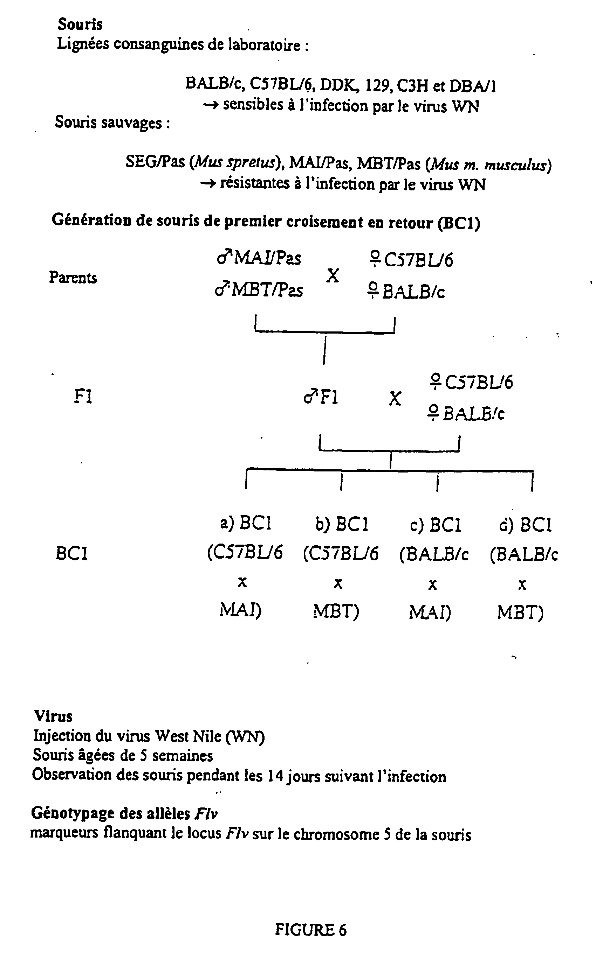

### a) Modèle d'analyse de la résistance à l'infection par les Flaviviridae (figure 6)

Des souris mâles des lignées résistantes MAI/Pas et MBT/Pas sont croisées avec des souris femelles des lignées sensibles C57BL/6 et BALB/c. Les souris mâles de la génération F1 sont croisées en retour avec des souris femelles des lignées sensibles C57BL/6 et BALB/c pour donner une génération de souris de premier croisement en retour (BC1).

Des souris BC1 âgées de 5 semaines sont inoculées par voie intrapéritonéale avec la souche IS-98-ST1, préparée selon le protocole décrit à l'exemple 1, dans les conditions décrites à l'exemple 2.

Les animaux sont observés tous les jours et les taux de mortalité et de survie sont déterminés 14 jours après l'infection.

### b) génotypage des allèles Flv

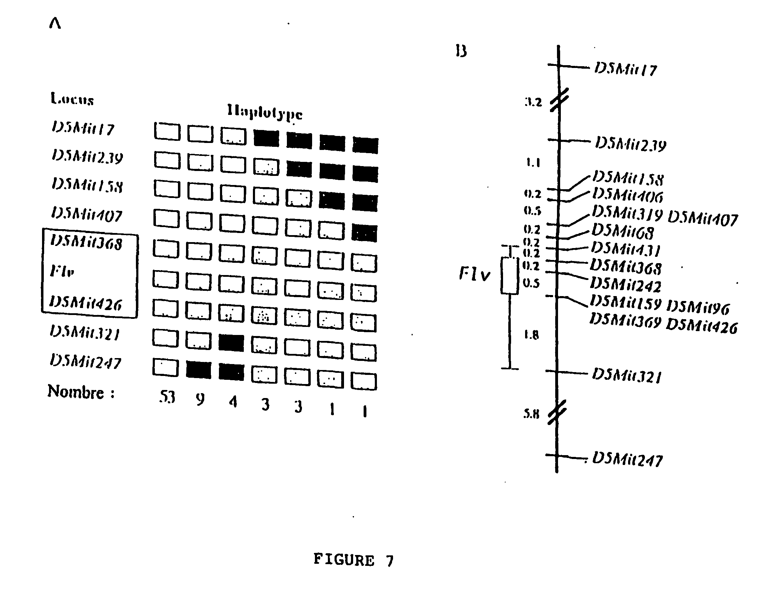

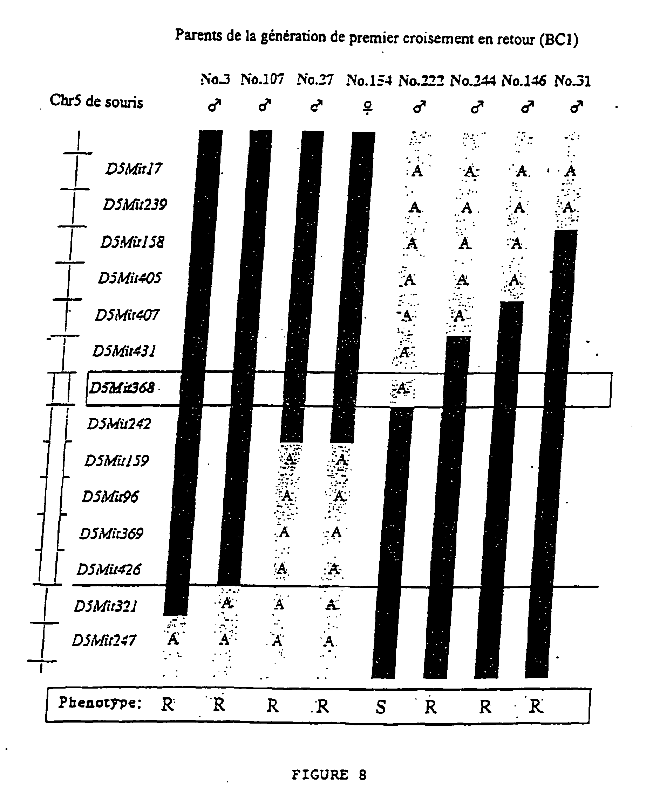

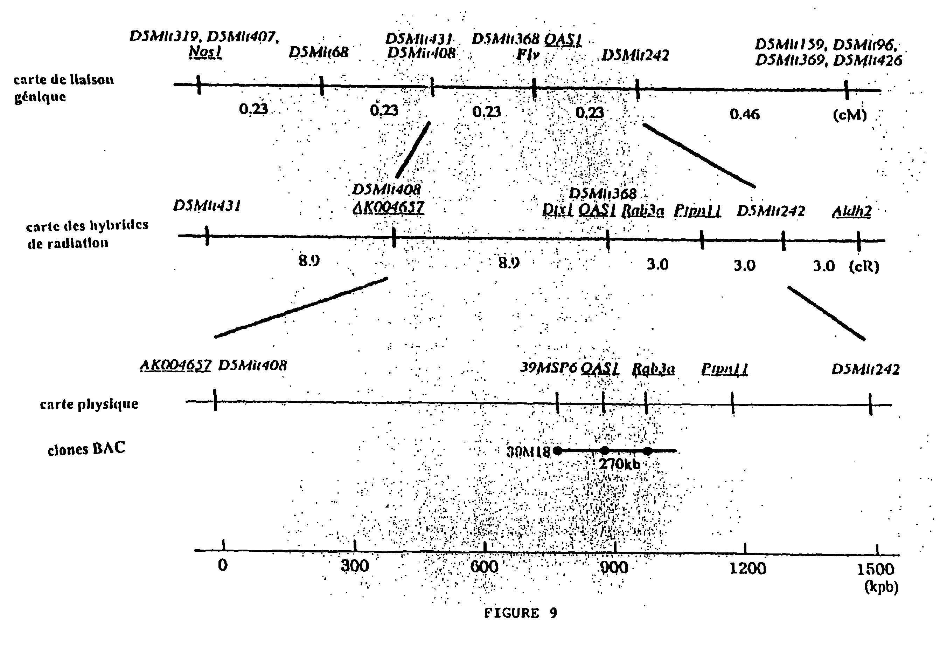

Les allèles *Flv* des individus BC1 ont été cartographiés par PCR génomique à l'aide d'amorces spécifiques de 16 microsatellites du chromosome 5 (Catalogue Research Genetics) entourant le locus *Flv* (figures 7-9), selon les techniques courantes de biologie moléculaire en utilisant les protocoles standards tels que ceux décrits dans Current Prolocols in Molecular Biology (Frederick M. AUSUBEL,2000, Wiley and son Inc, Library of Congress, USA).

### 2) Résultats

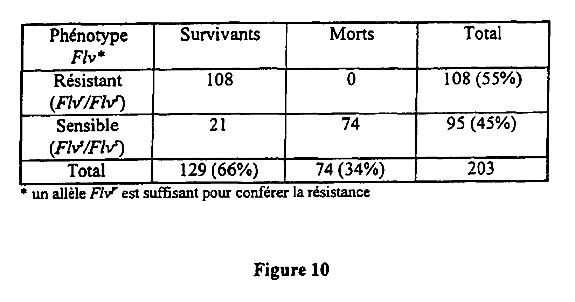

L'analyse de la distribution des allèles *Flv* chez les souris BC1 sensibles et résistantes à l'infection par la souche IS-98ST1 montre qu'un allèle *Flv*^{r} est suffisant pour conférer la résistance à l'infection (figure 10). Les résultats montrent également que dans ce modèle il existe une corrélation parfaite entre le phénotype résistant et la présence de l'allèle *Flv*^{r} et une corrélation presque parfaite entre le phénotype sensible et l'absence de l'allèle *Flv*^{r} (figure 10).

Le génotypage des allèles *Flv* montre que le locus *Flv* est localisé dans une région de 0.2 cM contenant le gène OAS 1 (figures 7-9).

## Revendications

1. Souche isolée du virus West-Nile, **caractérisée en ce que** son génome est constitué par la séquence SEQ ID NO :1.

2. Molécule d'acide nucléique, **caractérisée en ce qu'**elle est sélectionnée dans le groupe constitué par la séquence SEQ ID NO:1 et la séquence complémentaire anti-sens de la séquence SEQ ID NO :1.

3. Fragment de la molécule d'acide nucléique de séquence SEQ ID NO :1, **caractérisé en ce qu'**il est choisi dans le groupe constitué par les amorces comprenant au moins 15 nucléotides de ladite séquence situés entre 10 et 100 nucléotides en amont ou en aval de l'un des codons correspondant aux positions suivantes dans la séquence nucléotidique SEQ ID NO:1:
- codon Asparagine en positions 2518-2520 (position 17 de la séquence en acides aminés de la protéine NS1),
- codon Arginine en positions 4018-4020 (position 164 de la séquence en acides aminés de la protéine NS2A),
- codons Glycine en positions 4462-4464 et acide Glutamique en positions 4465-4467 (positions 82 et 83 de la séquence en acides aminés de la protéine NS2B),
- codons Proline en positions 6097-6099 et acide Glutamique en positions 6172-6174 (positions 496 et 521 de la séquence en acides aminés de la protéine NS3), et
- codons Serine en positions 7840-7842, Asparagine en positions 8518-8520 et Alanine en positions 8794-8796 (positions 54, 280 et 372 de la séquence en acides aminés de la protéine NS5).

4. Fragment de la molécule d'acide nucléique de séquence SEQ ID NO :1, **caractérisé en ce qu'**il est choisi dans le groupe constitué par les fragments, de préférence entre 50 et 200 nucléotides, amplifiés en utilisant les amorces selon la revendication 3.

5. Vecteur recombinant, **caractérisé en ce qu'**il comprend une molécule d'acide nucléique selon l'une quelconque des revendications 2 à 4.

6. Cellule eucaryote isolée, **caractérisée en ce qu'**elle est transformée par une molécule d'acide nucléique selon l'une quelconque des revendications 2 à 4, un vecteur selon la revendication 5 ou une souche neurovirulente du virus West-Nile selon la revendication 1.

7. Protéine ou peptide, **caractérisé en ce qu'**il est codé par une molécule d'acide nucléique selon l'une quelconque des revendications 2 à 4.

8. Utilisation de la souche selon la revendication 1 pour l'obtention d'un anticorps polyclonal, par immunisation d'un mammifère non-humain.

9. Utilisation selon la revendication 8, **caractérisée en ce que** ledit mammifère non-humain est une souris homozygote pour l'allèle *Flv^{r}* de résistance à l'infection par les virus de la famille des *Flaviviridae.*

10. Utilisation d'un vecteur recombinant selon la revendication 5 ou bien d'une protéine ou d'un peptide, selon la revendication 7 pour l'obtention d'un anticorps polyclonal ou monoclonal par immunisation d'un mammifère non-humain.

11. Modèle d'étude non humain de la sensibilité/résistance à l'infection par un virus de la famille des *Flaviviridae,* **caractérisé en ce qu'**il comprend au moins une souche neurovirulente du virus West-Nile selon la revendication 1.

12. Modèle d'étude selon la revendication 11, **caractérisé en ce qu'**il comprend en outre une souris homozygote pour l'allèle *Flv^{r}* ou *FIv^{s}.*

13. Procédé de détection d'une infection à *Flaviviridae,* notamment du virus West Nile, **caractérisé en ce qu'**il comprend :
- l'amplification des ARN issus d'un échantillon biologique à tester en utilisant comme amorces des molécules d'acide nucléique selon la revendication 3, et
- le séquençage du produit d'amplification obtenu.

14. Procédé de criblage de gènes cellulaires impliqués dans la résistance d'un mammifère à l'infection par un virus de la famille des *Flaviviridae,* **caractérisé en ce qu'**il comprend les étapes suivantes :
- mise en culture de cellules dérivées d'un hôte (humain ou non-humain) sélectionné pour sa résistance ou sa sensibilité à l'infection par un *Flaviviridae,*
- infection *in vitro* desdites cellules par la souche neurovirulente du virus West-Nile selon la revendication 1,
- détection de gènes exprimés de manière différentielle dans lesdites cellules infectées.

15. Utilisation du modèle selon la revendication 11 ou la revendication 12, pour le tri de molécules actives contre une infection virale due à un virus de la famille des *Flaviviridae.*

16. Procédé de tri de molécules actives contre une infection par un Flavivirus, **caractérisé par** :
- la mise en contact d'une culture de cellules eucaryotes, issues d'un mammifère sensible à l'infection à un *Flaviviridae* avec une suspension virale de la souche selon la revendication 1, en présence ou en l'absence de la molécule à tester et
- détection de la réplication du virus.

17. Variant de la souche virale selon la revendication 1, **caractérisée en ce que** son génome comprend au moins une mutation seulement dans la séquence nucléotidique correspondant à la protéine NS5.

18. Molécule d'acide nucléique, **caractérisée en ce qu'**elle est sélectionnée dans le groupe constitué par les séquences SEQ ID NO :3-11.

19. Procédé de détection d'une infection à virus West Nile, **caractérisé en ce qu'**il comprend :
- l'amplification des ARNs ou ADNs issus d'un échantillon biologique à tester en utilisant comme amorces des molécules d'acides nucléiques, selon la revendication 3,
- la mise en évidence du produit obtenu.

20. Coffret de détection d'une infection par un flavivirus comprenant au moins : des fragments de la molécule d'acide nucléique de SEQ ID NO : 1 tels que définis à la revendication 3.
- des réactifs pour visualiser la réaction d'hybridation entre lesdites molécules d'acides nucléiques et l'ADN ou l'ARN présent dans l'échantillon à tester.

## Claims

1. An isolated strain of the West Nile virus, **characterized in that** its genome consists of the sequence SEQ ID No. 1.

2. A nucleic acid molecule, **characterized in that** it is selected from the group consisting of the sequence SEQ ID No. 1, and the antisense sequence complementary to the sequence SEQ ID No. 1.

3. A fragment of the nucleic acid molecule of the sequence SEQ ID No. 1, **characterized in that** it is chosen from the group consisting of the primers comprising at least 15 nucleotides of said sequence located between 10 and 100 nucleotides upstream or downstream of one of the codons corresponding to the following positions in the nucleotide sequence SEQ ID No. 1:
- asparagine codon at positions 2518-2520 (position 17 of the amino acid sequence of the NS 1 protein),
- arginine codon at positions 4018-4020 (position 164 of the amino acid sequence of the NS2A protein),
- glycine codon at positions 4462-4464 and glutamic acid codon at positions 4465-4467 (positions 82 and 83 of the amino acid sequence of the NS2B protein),
- proline codon at positions 6097-6099 and glutamic acid codon at positions 6172-6174 (positions 496 and 521 of the amino acid sequence of the NS3 protein), and
- serine codon at positions 7840-7842, asparagine codon at positions 8518-8520 and alanine codon at positions 8794-8796 (position 54, 280 and 372 of the amino acid sequence of the NS5 protein).

4. A fragment of the nucleic acid molecule of the sequence SEQ ID No. 1, **characterized in that** it is chosen from the group consisting of the fragments, preferably of between 50 and 200 nucleotides, amplified using the primers as claimed in claim 3.

5. A recombinant vector, **characterized in that** it comprises a nucleic acid molecule as claimed in any one of claims 2 to 4.

6. An isolated eukaryotic cell, **characterized in that** it is transformed with a nucleic acid molecule as claimed in any one of claims 2 to 4, a vector as claimed in claim 5 or a neurovirulent strain of the West Nile virus as claimed in claim 1.

7. A protein or peptide, **characterized in that** it is encoded by a nucleic acid molecule as claimed in any one of claims 2 to 4.

8. A use of the strain as claimed in claim 1 for obtaining a polyclonal antibody by immunizing a non-human mammal.

9. The use according to claim 8, **characterized in that** said non-human mammal is a mouse homozygous for the Flv^{r} allele for resistance to infection with viruses of the family *Flaviviridae.*

10. A use of a recombinant vector as claimed in claim 5 or a protein or a peptide as claimed in claim 7 for obtaining a polyclonal or monoclonal antibody by immunizing a non-human mammal.

11. A non-human model for studying sensitivity/resistance to infection with a virus of the family *Flaviviridae,* **characterized in that** it comprises at least one neurovirulent strain of the West Nile virus as claimed in claim 1.

12. The study model as claimed in claim 11, **characterized in that** it also comprises a mouse homozygous for the Flv^{r} or Flv^{s} allele.

13. A method for detecting *Flaviviridae* infection, in particular a West Nile virus infection, **characterized in that** it comprises:
- amplifying the RNAs derived from a biological sample to be tested, using, as primers, nucleic acid molecules as claimed in claim 3, and
- sequencing the amplification product obtained.

14. A method for screening cellular genes involved in the resistance of a mammal to infection with a virus of the family *Flaviviridae,* **characterized in that** it comprises the following steps:
- culturing cells derived from a host (human or non-human) selected for its resistance or its sensitivity to infection with a *Flaviviridae,*
- infecting *in vitro* said cells with the neurvirulent strain of the West Nile virus as claimed in claim 1,
- detecting genes expressed differentially in said infected cells.

15. The use of the model as claimed in claim 11 or claim 12, for sorting molecules which are active against a viral infection due to a virus of the family *Flaviviridae.*

16. A method for sorting molecules which are active against infection with a Flavivirus, **characterized by**:
- bringing a culture of eukaryotic cells derived from a mammal sensitive to infection with a *Flaviviridae* into contact with a viral suspension of the strain as claimed in claim 1, in the presence or absence of the molecule to be tested, and
- detecting the replication of the virus.

17. A variant of the viral strain as claimed in claim 1, **characterized in that** its genome comprises at least one mutation in the nucleotide sequence corresponding to the NS5 protein only.

18. Nucleic acid molecule, **characterized in that** it is selected from the group consisting of the sequences SEQ ID Nos. 3-11.

19. A method for detecting a West Nile infection, **characterized in that** it comprises:
- amplifying the RNAs or DNAs derived from a biological sample to be tested, using as primers, nucleic acid molecules as claimed in claim 3,
- detecting the obtained product.

20. Kit for detecting a flavivirus infection, comprising at least:
- fragments of the nucleic acid molecule of the sequence SEQ ID No. 1 as defined in claim 3,
- reagents useful to visualize the hybridization reaction between said nucleic acid molecules and the RNA or DNA present in the biological sample to be tested.

## Patentansprüche

1. Isolierter Stamm des West-Nil-Virus, **dadurch gekennzeichnet, dass** sein Genom von der Sequenz SEQ ID Nr. 1 gebildet wird.

2. Nukleinsäuremolekül, **dadurch gekennzeichnet, dass** es ausgewählt ist aus der Gruppe, bestehen aus der Sequenz SEQ ID Nr. 1 und der komplementären Antisense-Sequenz der Sequenz SEQ ID Nr. 1.

3. Fragment des Nukleinsäuremoleküls der Sequenz SEQ ID Nr. 1, **dadurch gekennzeichnet, dass** es ausgewählt ist aus der Gruppe, bestehend aus den Sonden, die wenigstens 15 Nukleotide der besagten, zwischen 10 und 100 Nukleotiden gelegenen Sequenz strangaufwärts oder strangabwärts von einem der Codons umfassen, die den folgenden Positionen in der Nukleotidsequenz SEQ ID Nr. 1 entsprechen:
- Asparagincodon in Positionen 2518-2520 (Position 17 der Aminosäuresequenz des NS 1-Proteins),
- Arginincodon in Positionen 4018-4020 (Position 164 der Aminosäuresequenz des NS2A-Proteins),
- Glycincodon in Positionen 4462-4464 und Glutaminsäurecodon in Positionen 4465-4467 (Positionen 82 und 83 der Aminosäuresequenz des NS2B-Proteins),
- Prolincodon in Positionen 6097-6099 und Glutaminsäurecodon in Positionen 6172-6174 (Positionen 496 und 521 der Aminosäuresequenz des NS3-Proteins), und
- Serincodon in Positionen 7840-7842, Asparagincodon in Positionen 8518-8520 und Alanincodon in Positionen 8794-8796 (Positionen 54, 280 und 372 der Aminosäuresequenz des NS5-Proteins).

4. Fragment des Nukleinsäuremoleküls der Sequenz SEQ ID Nr. 1, **dadurch gekennzeichnet, dass** es ausgewählt ist aus der Gruppe, bestehend aus den Fragmenten, bevorzugt zwischen 20 und 200 Nukleotiden, die unter Verwenden der Sonden nach Anspruch 3 amplifiziert wurden.

5. Rekombinanter Vektor, **dadurch gekennzeichnet, dass** er ein Nukleinsäuremolekül nach einem der Ansprüche 2 bis 4 umfasst.

6. Isolierte eukaryotische Zelle, **dadurch gekennzeichnet, dass** sie mit einem Nukleinsäuremolekül nach einem der Ansprüche 2 bis 4, einem Vektor nach Anspruch 5 oder einem neurovirulenten Stamm des West-Nil-Virus nach Anspruch 1 transformiert ist.

7. Protein oder Peptid, **dadurch gekennzeichnet, dass** es von einem Nukleinsäuremolekül nach einem der Ansprüche 2 bis 4 codiert wird.

8. Verwendung eines Stamms nach Anspruch 1, zum Erhalt eines polyklonalen Antikörpers durch Immunisierung eines nicht-menschlichen Säugetiers.

9. Verwendung nach Anspruch 8, **dadurch gekennzeichnet, dass** das nichtmenschliche Säugetier eine für das Flv^{r}-Allel homozygote Maus ist, mit einer Resistenz gegen Infektion mit den Viren der Familie der *Flaviviridae.*

10. Verwendung eines rekombinanten Vektors nach Anspruch 5 oder eines Proteins oder eines Peptids nach Anspruch 7, zum Erhalt eines polyklonalen oder monoklonalen Antikörpers durch Immunisierung eines nicht-menschlichen Säugetiers.

11. Nicht-menschliches Untersuchungsmodel der Sensibilität/Resistenz gegenüber einem Virus der Familie der *Flaviviridae,* **dadurch gekennzeichnet, dass** es wenigstens einen neurovirulenten Stamm des West-Nil-Virus nach Anspruch 1 umfasst.

12. Untersuchungsmodel nach Anspruch 11, **dadurch gekennzeichnet, dass** es unter anderem eine für das Flv^{r}- oder Flv^{s}-Allel homozygote Maus umfasst.

13. Verfahren zum Detektieren einer *Flaviviridae* Infektion, insbesondere mit dem West-Nil-Virus, **dadurch gekennzeichnet, dass** es umfasst:
- Amplifizieren der aus einer biologischen Untersuchungsprobe stammenden RNA unter Verwenden von Nukleinsäuremolekülen nach Anspruch 3 als Sonden, und
- Sequenzieren des erhaltenen Amplifikationsprodukts.

14. Screening-Verfahren für zelluläre Gene, die in der Resistenz eines Säugetiers gegen eine Infektion mit einem Virus der Familie der *Flaviviridae* involviert sind, **dadurch gekennzeichnet, dass** es die folgenden Schritte umfasst:
- Kultivieren von von einem Wirt abstammenden Zellen (menschlich oder nichtmenschlich), der wegen seiner Resistenz oder Sensitivität gegen eine Infektion mit *Flaviviridae* ausgewählt wurde,
- *in vitro* Infizieren der Zellen mit einem neurovirulenten Stamm des West-Nil-Virus nach Anspruch 1,
- Detektieren der differentiell exprimierten Gene in den infizierten Zellen.

15. Verwendung eines Modells nach Anspruch 11 oder Anspruch 12, zum Aussortieren von Molekülen, die gegen eine durch einen Virus der Familie der *Flaviviridae* verschuldete virale Infektion aktiv sind.

16. Verfahren zum Aussortieren von Molekülen, die gegen eine Infektion mit dem Flavivirus aktiv sind, **gekennzeichnet durch**:
- In Kontakt bringen einer Kultur eukaryotischer Zellen, die von einem Säugetier stammt, das für eine Infektion mit *Flaviviridae* empfindlich ist, mit einer viralen Suspension des Stamms nach Anspruch 1, in Anwesenheit oder Abwesenheit des zu untersuchenden Moleküls, und
- Detektion der Virusreplikation.

17. Variante des viralen Stamms nach Anspruch 1, **dadurch gekennzeichnet, dass** ihr Genom wenigstens eine Mutation nur in der Nukleotidsequenz umfasst, die dem NS5-Protein entspricht.

18. Nukleinsäuremolekül, **dadurch gekennzeichnet, dass** es ausgewählt ist aus der Gruppe, bestehend aus den Sequenzen SEQ ID Nr. 3-11.

19. Verfahren zum Detektieren einer Infektion mit dem West-Nil-Virus, **dadurch gekennzeichnet, dass** es umfasst:
- Amplifizieren der aus einer biologischen Untersuchungsprobe stammenden RNA oder DNA, unter Verwenden von Nukleinsäuremolekülen nach Anspruch 3 als Sonden,
- Aufzeigen des erhaltenen Produkts.

20. Detektionsgerät für eine Infektion mit dem Flavivirus, umfassend wenigstens:
- Fragmente des Nukleinsäuremoleküls der Sequenz SEQ ID Nr. 1, wie sie in Anspruch 3 definiert sind,
- Reagenzien zum Visualisieren der Hybridisierungsreaktion zwischen den Nukleinsäuremolekülen und der in der Untersuchungsprobe vorliegenden DNA oder RNA.


### LISTE DE SEQUENCES

<110> INSTITUT PASTEUR
   KIMRON VETERINARY INSTITUTE
   DESPRES Philippe
   DEUBEL Vincent
   GUENET Jean-Louis
   DROUET Marie-Thérèse
   MALKINSON Mertyn
   BANET Caroline
   FRENKIEL Marie-Pascale
   COURAGEOT Marie-Pierre
   COULILABY Fasséli
   CATTEAU Adeline
   FLAMAND Marie
   WEBER Patrick
   CECCALDI Pierre-Emmanuel
<120> SOUCHE NEUROVIRULENTE DU VIRUS WEST NILE ET SES APPLICATIONS
<130> 226CAS93EXT
<140>
   <141>
<160> 11
<170> PatentIn Ver. 2.1
<210> 1
   <211> 11029
   <212> ARN
   <213> Flavivirus sp.
<220>
   <221> CDS
   <222> (97)..(10395)
<400> 1
   agtagttcgc ctgtgtgagc tgacaaactt agtagtgttt gtgaggatta acaacaatta 60
   acacagtgcg agctgtttct tagcacgaag atctcg atg tct aag aaa cca gga   114
                                           Met Ser Lys Lys Pro Gly
                                             1               5
   ggg ccc ggc aag agc cgg gct gtc aat atg cta aaa cgc gga atg ccc   162
   Gly Pro Gly Lys Ser Arg Ala Val Asn Met Leu Lys Arg Gly Met Pro
               10                  15                  20
   cgc gtg ttg tcc ttg att gga ctg aag agg gct atg ttg agc ctg atc   210
   Arg Val Leu Ser Leu Ile Gly Leu Lys Arg Ala Met Leu Ser Leu Ile
           25                  30                  35
   gac ggc aag ggg cca ata cga ttt gtg ttg gct ctc ttg gcg ttc ttc   258
   Asp Gly Lys Gly Pro Ile Arg Phe Val Leu Ala Leu Leu Ala Phe Phe
       40                  45                  50
   agg ttc aca gca att gct ccg acc cga gca gtg ctg gat cga tgg aga   306
   Arg Phe Thr Ala Ile Ala Pro Thr Arg Ala Val Leu Asp Arg Trp Arg
   55                  60                  65                  70
   ggt gtg aat aaa caa aca gcg atg aaa cac ctt ctg agt ttt aag aag    354
   Gly Val Asn Lys Gln Thr Ala Met Lys His Leu Leu Ser Phe Lys Lys
                   75                  80                  85
   gaa cta ggg acc ttg acc agt gct atc aat cgg cgg agc tca aaa caa    402
   Glu Leu Gly Thr Leu Thr Ser Ala Ile Asn Arg Arg Ser Ser Lys Gln
               90                  95                  100
   aag aaa aga gga gga aag acc gga att gca gtc atg att ggc ctg atc    450
   Lys Lys Arg Gly Gly Lys Thr Gly Ile Ala Val Met Ile Gly Leu Ile
           105                 110                 115
   gcc agc gta gga gca gtt acc ctc tct aac ttc caa ggg aag gtg atg    498
   Ala Ser Val Gly Ala Val Thr Leu Ser Asn Phe Gln Gly Lys Val Met
       120                 125                 130
   atg acg gta aat gct act gac gtc aca gat gtc atc acg att cca aca    546
   Met Thr Val Asn Ala Thr Asp Val Thr Asp Val Ile Thr Ile Pro Thr
   135                 140                 145                 150
   gct gct gga aag aac cta tgc att gtc aga gca atg gat gtg gga tac    594
   Ala Ala Gly Lys Asn Leu Cys Ile Val Arg Ala Met Asp Val Gly Tyr
                   155                 160                 165
   atg tgc gat gat act atc act tat gaa tgc cca gtg ctg tcg gct ggt    642
   Met Cys Asp Asp Thr Ile Thr Tyr Glu Cys Pro Val Leu Ser Ala Gly
               170                 175                 180
   aat gat cca gaa gac atc gac tgt tgg tgc aca aag tca gca gtc tac    690
   Asn Asp Pro Glu Asp Ile Asp Cys Trp Cys Thr Lys Ser Ala Val Tyr
           185                 190                 195
   gtc agg tat gga aga tgc acc aag aca cgc cac tca aga cgc agt cgg    738
   Val Arg Tyr Gly Arg Cys Thr Lys Thr Arg His Ser Arg Arg Ser Arg
       200                 205                 210
   agg tca ctg aca gtg cag aca cac gga gaa agc act cta gcg aac aag    786
   Arg Ser Leu Thr Val Gln Thr His Gly Glu Ser Thr Leu Ala Asn Lys
   215                 220                 225                 230
   aag ggg gct tgg atg gac agc acc aag gcc aca agg tat ttg gta aaa    834
   Lys Gly Ala Trp Met Asp Ser Thr Lys Ala Thr Arg Tyr Leu Val Lys
                   235                 240                 245
   aca gaa tca tgg atc ttg agg aac cct gga tat gcc ctg gtg gca gcc    882
   Thr Glu Ser Trp Ile Leu Arg Asn Pro Gly Tyr Ala Leu Val Ala Ala
               250                 255                 260
   gtc att ggt tgg atg ctt ggg agc aac acc atg cag aga gtt gtg ttt    930
   Val Ile Gly Trp Met Leu Gly Ser Asn Thr Met Gln Arg Val Val Phe
           265                 270                 275
   gtc gtg cta ttg ctt ttg gtg gcc cca gct tac agc ttt aac tgc ctt    978
   Val Val Leu Leu Leu Leu Val Ala Pro Ala Tyr Ser Phe Asn Cys Leu
       280                 285                 290
   gga atg agc aac aga gac ttc ttg gaa gga gtg tct gga gca aca tgg   1026
   Gly Met Ser Asn Arg Asp Phe Leu Glu Gly Val Ser Gly Ala Thr Trp
   295                 300                 305                 310
   gtg gat ttg gtt ctc gaa ggc gac agc tgc gtg act atc atg tct aag   1074
   Val Asp Leu Val Leu Glu Gly Asp Ser Cys Val Thr Ile Met Ser Lys
                   315                 320                 325
   gac aag cct acc atc gat gtg aag atg atg aat atg gag gcg gcc aac   1122
   Asp Lys Pro Thr Ile Asp Val Lys Met Met Asn Met Glu Ala Ala Asn
               330                 335                 340
   ctg gca gag gtc cgc agt tat tgc tat ttg gct acc gtc agc gat ctc   1170
   Leu Ala Glu Val Arg Ser Tyr Cys Tyr Leu Ala Thr Val Ser Asp Leu
           345                 350                 355
   tcc acc aaa gct gcg tgc ccg acc atg gga gaa gct cac aat gac aaa   1218
   Ser Thr Lys Ala Ala Cys Pro Thr Met Gly Glu Ala His Asn Asp Lys
       360                 365                 370
   cgt gct gac cca gct ttt gtg tgc aga caa gga gtg gtg gac agg ggc   1266
   Arg Ala Asp Pro Ala Phe Val Cys Arg Gln Gly Val Val Asp Arg Gly
   375                 380                 385                 390
   tgg ggc aac ggc tgc gga cta ttt ggc aaa gga agc att gac aca tgc   1314
   Trp Gly Asn Gly Cys Gly Leu Phe Gly Lys Gly Ser Ile Asp Thr Cys
                   395                 400                 405
   gcc aaa ttt gcc tgc tct acc aag gca ata gga aga acc atc ttg aaa   1362
   Ala Lys Phe Ala Cys Ser Thr Lys Ala Ile Gly Arg Thr Ile Leu Lys
               410                 415                 420
   gag aat atc aag tac gaa gtg gcc att ttt gtc cat gga cca act act   1410
   Glu Asn Ile Lys Tyr Glu Val Ala Ile Phe Val His Gly Pro Thr Thr
           425                 430                 435
   gtg gag tcg cac gga aac tac tcc aca cag gtt gga gcc act cag gca   1458
   Val Glu Ser His Gly Asn Tyr Ser Thr Gln Val Gly Ala Thr Gln Ala
       440                 445                 450
   ggg aga ttc agc atc act cct gcg gcg cct tca tac aca cta aag ctt   1506
   Gly Arg Phe Ser Ile Thr Pro Ala Ala Pro Ser Tyr Thr Leu Lys Leu
   455                 460                 465                 470
   gga gaa tat gga gag gtg aca gtg gac tgt gaa cca cgg tca ggg att   1554
   Gly Glu Tyr Gly Glu Val Thr Val Asp Cys Glu Pro Arg Ser Gly Ile
                   475                 480                 485
   gac acc aat gca tac tac gtg atg act gtt gga aca aag acg ttc ttg   1602
   Asp Thr Asn Ala Tyr Tyr Val Met Thr Val Gly Thr Lys Thr Phe Leu
               490                 495                 500
   gtc cat cgt gag tgg ttc atg gac ctc aac ctc cct tgg agc agt gct   1650
   Val His Arg Glu Trp Phe Met Asp Leu Asn Leu Pro Trp Ser Ser Ala
           505                 510                 515
   gga agt act gtg tgg agg aac aga gag acg tta atg gag ttt gag gaa   1698
   Gly Ser Thr Val Trp Arg Asn Arg Glu Thr Leu Met Glu Phe Glu Glu
       520                 525                 530
   cca cac gcc acg aag cag tct gtg ata gca ttg ggc tca caa gag gga   1746
   Pro His Ala Thr Lys Gln Ser Val Ile Ala Leu Gly Ser Gln Glu Gly
   535                 540                 545                 550
   gct ctg cat caa gct ttg gct gga gcc att cct gtg gaa ttt tca agc    1794
   Ala Leu His Gln Ala Leu Ala Gly Ala Ile Pro Val Glu Phe Ser Ser
                   555                 560                 565
   aac act gtc aag ttg acg tcg ggt cat ttg aag tgt aga gtg aag atg    1842
   Asn Thr Val Lys Leu Thr Ser Gly His Leu Lys Cys Arg Val Lys Met
               570                 575                 580
   gaa aaa ttg cag ttg aag gga aca acc tat ggc gtc tgt tca aag gct    1890
   Glu Lys Leu Gln Leu Lys Gly Thr Thr Tyr Gly Val Cys Ser Lys Ala
           585                 590                 595
   ttc aag ttt ctt ggg act ccc gca gac aca ggt cac ggc act gtg gtg    1938
   Phe Lys Phe Leu Gly Thr Pro Ala Asp Thr Gly His Gly Thr Val Val
       600                 605                 610
   ttg gaa ttg cag tac act ggc acg gat gga cct tgc aaa gtt cct atc    1986
   Leu Glu Leu Gln Tyr Thr Gly Thr Asp Gly Pro Cys Lys Val Pro Ile
   615                 620                 625                 630
   tcg tca gtg gct tca ttg aac gac cta acg cca gtg ggc aga ttg gtc    2034
   Ser Ser Val Ala Ser Leu Asn Asp Leu Thr Pro Val Gly Arg Leu Val
                   635                 640                 645
   act gtc aac cct ttt gtt tca gtg gcc acg gcc aac gct aag gtc ctg    2082
   Thr Val Asn Pro Phe Val Ser Val Ala Thr Ala Asn Ala Lys Val Leu
               650                 655                 660
   att gaa ttg gaa cca ccc ttt gga gac tca tac ata gtg gtg ggc aga    2130
   Ile Glu Leu Glu Pro Pro Phe Gly Asp Ser Tyr Ile Val Val Gly Arg
           665                 670                 675
   gga gaa caa cag att aat cac cat tgg cac aag tct gga agc agc att    2178
   Gly Glu Gln Gln Ile Asn His His Trp His Lys Ser Gly Ser Ser Ile
       680                 685                 690
   ggc aaa gcc ttt aca acc acc ctc aaa gga gcg cag aga cta gcc gct    2226
   Gly Lys Ala Phe Thr Thr Thr Leu Lys Gly Ala Gln Arg Leu Ala Ala
   695                 700                 705                 710
   cta gga gac aca gct tgg gac ttt gga tca gtt gga ggg gtg ttc acc    2274
   Leu Gly Asp Thr Ala Trp Asp Phe Gly Ser Val Gly Gly Val Phe Thr
                   715                 720                 725
   tca gtt ggg aag gct gtc cat caa gtg ttc gga gga gca ttc cgc tca    2322
   Ser Val Gly Lys Ala Val His Gln Val Phe Gly Gly Ala Phe Arg Ser
               730                 735                 740
   ctg ttc gga ggc atg tcc tgg ata acg caa gga ttg ctg ggg gct ctc    2370
   Leu Phe Gly Gly Met Ser Trp Ile Thr Gln Gly Leu Leu Gly Ala Leu
           745                 750                 755
   ctg ttg tgg atg ggc atc aat gct cgt gat agg tcc ata gct ctc acg    2418
   Leu Leu Trp Met Gly Ile Asn Ala Arg Asp Arg Ser Ile Ala Leu Thr
       760                 765                 770
   ttt ctc gca gtt gga gga gtt ctg ctc ttc ctc tcc gtg aac gtg cac    2466
   Phe Leu Ala Val Gly Gly Val Leu Leu Phe Leu Ser Val Asn Val His
   775                 780                 785                 790
   gct gac act ggg tgt gcc ata gac atc agc cgg caa gag ctg aga tgt    2514
   Ala Asp Thr Gly Cys Ala Ile Asp Ile Ser Arg Gln Glu Leu Arg Cys
                   795                 800                 805
   gga aat gga gtg ttc ata cac aat gat gtg gag gct tgg atg gac cgg    2562
   Gly Asn Gly Val Phe Ile His Asn Asp Val Glu Ala Trp Met Asp Arg
               810                 815                 820
   tac aag tat tac cct gaa acg cca caa ggc cta gcc aag atc att cag    2610
   Tyr Lys Tyr Tyr Pro Glu Thr Pro Gln Gly Leu Ala Lys Ile Ile Gln
           825                 830                 835
   aaa gct cat aag gaa gga gtg tgc ggt cta cga tca gtt tcc aga ctg    2658
   Lys Ala His Lys Glu Gly Val Cys Gly Leu Arg Ser Val Ser Arg Leu
       840                 845                 850
   gag cat caa atg tgg gaa gca gtg aag gac gag ctg aac act ctt ttg    2706
   Glu His Gln Met Trp Glu Ala Val Lys Asp Glu Leu Asn Thr Leu Leu
   855                 860                 865                 870
   aag gag aat ggt gtg gac ctt agt gtc gtg gtt gag aaa cag gag gga    2754
   Lys Glu Asn Gly Val Asp Leu Ser Val Val Val Glu Lys Gln Glu Gly
                   875                 880                 885
   atg tac aag tca gca cct aaa cgc ctc acc gcc acc acg gaa aaa ttg    2802
   Met Tyr Lys Ser Ala Pro Lys Arg Leu Thr Ala Thr Thr Glu Lys Leu
               890                 895                 900
   gaa att ggc tgg aag gcc tgg gga aag agt att tta ttt gca cca gaa    2850
   Glu Ile Gly Trp Lys Ala Trp Gly Lys Ser Ile Leu Phe Ala Pro Glu
           905                 910                 915
   ctc gcc aac aac acc ttt gtg gtt gat ggt ccg gag acc aag gaa tgt    2898
   Leu Ala Asn Asn Thr Phe Val Val Asp Gly Pro Glu Thr Lys Glu Cys
       920                 925                 930
   ccg act cag aat cgc gct tgg aat agc tta gaa gtg gag gat ttt gga    2946
   Pro Thr Gln Asn Arg Ala Trp Asn Ser Leu Glu Val Glu Asp Phe Gly
   935                 940                 945                 950
   ttt ggt ctc acc agc act cgg atg ttc ctg aag gtc aga gag agc aac    2994
   Phe Gly Leu Thr Ser Thr Arg Met Phe Leu Lys Val Arg Glu Ser Asn
                   955                 960                 965
   aca act gaa tgt gac tcg aag atc att gga acg gct gtc aag aac aac    3042
   Thr Thr Glu Cys Asp Ser Lys Ile Ile Gly Thr Ala Val Lys Asn Asn
               970                 975                 980
   ttg gcg atc cac agt gac ctg tcc tat tgg att gaa agc agg ctc aat    3090
   Leu Ala Ile His Ser Asp Leu Ser Tyr Trp Ile Glu Ser Arg Leu Asn
           985                 990                 995
   gat acg tgg aag ctt gaa agg gca gtt ctg ggt gaa gtc aaa tca tgt    3138
   Asp Thr Trp Lys Leu Glu Arg Ala Val Leu Gly Glu Val Lys Ser Cys
       1000                1005                1010
   acg tgg cct gag acg cat acc ttg tgg ggc gat gga atc ctt gag agt    3186
   Thr Trp Pro Glu Thr His Thr Leu Trp Gly Asp Gly Ile Leu Glu Ser
   1015                1020                1025                1030
   gac ttg ata ata cca gtc aca ctg gcg gga cca cga agc aat cac aat    3234
   Asp Leu Ile Ile Pro Val Thr Leu Ala Gly Pro Arg Ser Asn His Asn
                   1035                1040                1045
   cgg aga cct ggg tac aag aca caa aac cag ggc cca tgg gac gaa ggc    3282
   Arg Arg Pro Gly Tyr Lys Thr Gln Asn Gln Gly Pro Trp Asp Glu Gly
               1050                1055                1060
   cgg gta gag att gac ttc gat tac tgc cca gga act acg gtc acc ctg    3330
   Arg Val Glu Ile Asp Phe Asp Tyr Cys Pro Gly Thr Thr Val Thr Leu
           1065                1070                1075
   agt gag agc tgc gga cac cgt gga cct gcc act cgc acc acc aca gag    3378
   Ser Glu Ser Cys Gly His Arg Gly Pro Ala Thr Arg Thr Thr Thr Glu
       1080                1085                1090
   agc gga aag ttg ata aca gat tgg tgc tgc agg agc tgc acc tta cca    3426
   Ser Gly Lys Leu Ile Thr Asp Trp Cys Cys Arg Ser Cys Thr Leu Pro
   1095                1100                1105                1110
   cca ctg cgc tac caa act gac agc ggc tgt tgg tat ggt atg gag atc    3474
   Pro Leu Arg Tyr Gln Thr Asp Ser Gly Cys Trp Tyr Gly Met Glu Ile
                   1115                1120                1125
   aga cca cag aga cat gat gaa aag acc ctc gtg cag tca caa gtg aat    3522
   Arg Pro Gln Arg His Asp Glu Lys Thr Leu Val Gln Ser Gln Val Asn
               1130                1135                1140
   gct tat aat gct gat atg att gac cct ttt cag ttg ggc ctt ctg gtc    3570
   Ala Tyr Asn Ala Asp Met Ile Asp Pro Phe Gln Leu Gly Leu Leu Val
           1145                1150                1155
   gtg ttc ttg gcc acc cag gag gtc ctt cgc aag agg tgg aca gcc aag    3618
   Val Phe Leu Ala Thr Gln Glu Val Leu Arg Lys Arg Trp Thr Ala Lys
       1160                1165                1170
   atc agc atg cca gct ata ctg att gct ctg cta gtc ctg gtg ttt ggg    3666
   Ile Ser Met Pro Ala Ile Leu Ile Ala Leu Leu Val Leu Val Phe Gly
   1175                1180                1185                1190
   ggc att act tac act gat gtg tta cgc tat gtc atc ttg gtg ggg gca    3714
   Gly Ile Thr Tyr Thr Asp Val Leu Arg Tyr Val Ile Leu Val Gly Ala
                   1195                1200                1205
   gct ttc gca gaa tct aat tcg gga gga gac gtg gta cac ttg gcg ctc    3762
   Ala Phe Ala Glu Ser Asn Ser Gly Gly Asp Val Val His Leu Ala Leu
               1210                1215                1220
   atg gcg acc ttc aag ata caa cca gtg ttt atg gtg gca tcg ttt ctc    3810
   Met Ala Thr Phe Lys Ile Gln Pro Val Phe Met Val Ala Ser Phe Leu
           1225                1230                1235
   aaa gcg aga tgg acc aac cag gag aac att ttg ttg atg ttg gcg gct    3858
   Lys Ala Arg Trp Thr Asn Gln Glu Asn Ile Leu Leu Met Leu Ala Ala
       1240                1245                1250
   gtt ttc ttt caa atg gct tat cac gat gcc cgc caa att ctg ctc tgg    3906
   Val Phe Phe Gln Met Ala Tyr His Asp Ala Arg Gln Ile Leu Leu Trp
   1255                1260                1265                1270
   gag atc cct gat gtg ttg aat tca ctg gcg gta gct tgg atg ata ctg   3954
   Glu Ile Pro Asp Val Leu Asn Ser Leu Ala Val Ala Trp Met Ile Leu
                   1275                1280                1285
   aga gcc ata aca ttc aca acg aca tca aat gtg gtc gtc ccg ctg cta   4002
   Arg Ala Ile Thr Phe Thr Thr Thr Ser Asn Val Val Val Pro Leu Leu
               1290                1295                1300
   gcc ctg cta aca ccc cgg ctg aga tgc ttg aat ctg gat gtg tac agg   4050
   Ala Leu Leu Thr Pro Arg Leu Arg Cys Leu Asn Leu Asp Val Tyr Arg
           1305                1310                1315
   ata ctg ctg ttg atg gtc gga ata ggc agc ttg atc agg gag aag agg   4098
   Ile Leu Leu Leu Met Val Gly Ile Gly Ser Leu Ile Arg Glu Lys Arg
       1320                1325                1330
   agt gca gct gca aaa aag aaa gga gca agt ctg cta tgc ttg gct cta   4146
   Ser Ala Ala Ala Lys Lys Lys Gly Ala Ser Leu Leu Cys Leu Ala Leu
   1335                1340                1345                1350
   gcc tca aca gga ctt ttc aac ccc atg atc ctt gct gct gga ctg att   4194
   Ala Ser Thr Gly Leu Phe Asn Pro Met Ile Leu Ala Ala Gly Leu Ile
                   1355                1360                1365
   gca tgt gat ccc aac cgt aaa cgc gga tgg ccc gca act gaa gtg atg   4242
   Ala Cys Asp Pro Asn Arg Lys Arg Gly Trp Pro Ala Thr Glu Val Met
               1370                1375                1380
   aca gct gtc ggc cta atg ttt gcc atc gtc gga ggg ctg gca gag ctt   4290
   Thr Ala Val Gly Leu Met Phe Ala Ile Val Gly Gly Leu Ala Glu Leu
           1385                1390                1395
   gac att gac tcc atg gcc att cca atg act atc gcg ggg ctc atg ttt   4338
   Asp Ile Asp Ser Met Ala Ile Pro Met Thr Ile Ala Gly Leu Met Phe
       1400                1405                1410
   gct gct ttc gtg att tct ggg aaa tca aca gat atg tgg att gag aga   4386
   Ala Ala Phe Val Ile Ser Gly Lys Ser Thr Asp Met Trp Ile Glu Arg
   1415                1420                1425                1430
   acg gcg gac att tcc tgg gaa agt gat gca gaa att aca ggc tcg agc   4434
   Thr Ala Asp Ile Ser Trp Glu Ser Asp Ala Glu Ile Thr Gly Ser Ser
                   1435                1440                1445
   gaa aga gtt gat gtt cgg ctt gat gat ggt gaa aac ttc cag ctc atg   4482
   Glu Arg Val Asp Val Arg Leu Asp Asp Gly Glu Asn Phe Gln Leu Met
               1450                1455                1460
   aat gat cca gga gca cct tgg aag ata tgg atg ctc aga atg gtc tgt   4530
   Asn Asp Pro Gly Ala Pro Trp Lys Ile Trp Met Leu Arg Met Val Cys
           1465                1470                1475
   ctc gcg att agt gcg tac acc ccc tgg gca atc ttg ccc tca gta gtt   4578
   Leu Ala Ile Ser Ala Tyr Thr Pro Trp Ala Ile Leu Pro Ser Val Val
       1480                1485                1490
   gga ttt tgg ata act ctc caa tac aca aag aga gga ggt gtg ttg tgg   4626
   Gly Phe Trp Ile Thr Leu Gln Tyr Thr Lys Arg Gly Gly Val Leu Trp
   1495                1500                1505                1510
   gac act ccc tca cca aag gag tac aaa aag ggg gac acg acc acc ggc 4674
   Asp Thr Pro Ser Pro Lys Glu Tyr Lys Lys Gly Asp Thr Thr Thr Gly
   1515 1520 1525
   gtc tac agg atc atg act cgt ggg ctg ctc ggc agt tat caa gca gga 4722
   Val Tyr Arg Ile Met Thr Arg Gly Leu Leu Gly Ser Tyr Gln Ala Gly
   1530 1535 1540
   gcg ggc gtg atg gtt gaa ggt gtt ttc cac acc ctt tgg cat aca aca 4770
   Ala Gly Val Met Val Glu Gly Val Phe His Thr Leu Trp His Thr Thr
   1545 1550 1555
   aaa gga gcc gct ttg atg agc gga gag ggc cgc ctg gac cca tac tgg 4818
   Lys Gly Ala Ala Leu Met Ser Gly Glu Gly Arg Leu Asp Pro Tyr Trp
   1560 1565 1570
   ggc agt gtc aag gag gat cga ctt tgt tac gga gga ccc tgg aaa ttg 4866
   Gly Ser Val Lys Glu Asp Arg Leu Cys Tyr Gly Gly Pro Trp Lys Leu
   1575 1580 1585 1590
   cag cac aag tgg aac ggg cag gat gag gtg cag atg att gtg gtg gaa 4914
   Gln His Lys Trp Asn Gly Gln Asp Glu Val Gln Met Ile Val Val Glu
   1595 1600 1605
   cct ggc aag aac gtt aag aac gtc cag acg aaa cca ggg gtg ttc aaa 4962
   Pro Gly Lys Asn Val Lys Asn Val Gln Thr Lys Pro Gly Val Phe Lys
   1610 1615 1620
   aca cct gaa gga gaa atc ggg gcc gtg act ttg gac ttc ccc act gga 5010
   Thr Pro Glu Gly Glu Ile Gly Ala Val Thr Leu Asp Phe Pro Thr Gly
   1625 1630 1635
   aca tca ggc tca cca ata gtg gac aaa aac ggt gat gtg att ggg ctt 5058
   Thr Ser Gly Ser Pro Ile Val Asp Lys Asn Gly Asp Val Ile Gly Leu
   1640 1645 1650
   tat ggc aat gga gtc ata atg ccc aac ggc tca tac ata agc gcg ata 5106
   Tyr Gly Asn Gly Val Ile Met Pro Asn Gly Ser Tyr Ile Ser Ala Ile
   1655 1660 1665 1670
   gtg cag ggt gaa agg atg gat gag cca atc cca gcc gga ttc gaa cct 5154
   Val Gln Gly Glu Arg Met Asp Glu Pro Ile Pro Ala Gly Phe Glu Pro
   1675 1680 1685
   gag atg ctg agg aaa aaa cag atc act gta ctg gat ctc cat ccc ggc 5202
   Glu Met Leu Arg Lys Lys Gln Ile Thr Val Leu Asp Leu His Pro Gly
   1690 1695 1700
   gcc ggt aaa aca agg agg att ctg cca cag atc atc aaa gag gcc ata 5250
   Ala Gly Lys Thr Arg Arg Ile Leu Pro Gln Ile Ile Lys Glu Ala Ile
   1705 1710 1715
   aac aga aga ctg aga aca gcc gtg cta gca cca acc agg gtt gtg gct 5298
   Asn Arg Arg Leu Arg Thr Ala Val Leu Ala Pro Thr Arg Val Val Ala
   1720 1725 1730
   gct gag atg gct gaa gca ctg aga gga ctg ccc atc cgg tac cag aca 5346
   Ala Glu Met Ala Glu Ala Leu Arg Gly Leu Pro Ile Arg Tyr Gln Thr
   1735 1740 1745 1750
   tcc gca gtg ccc aga gaa cat aat gga aat gag att gtt gat gtc atg    5394
   Ser Ala Val Pro Arg Glu His Asn Gly Asn Glu Ile Val Asp Val Met
                   1755                1760                1765
   tgt cat gct acc ctc acc cac agg ctg atg tct cct cac agg gtg ccg    5442
   Cys His Ala Thr Leu Thr His Arg Leu Met Ser Pro His Arg Val Pro
               1770                1775                1780
   aac tac aac ctg ttc gtg atg gat gag gct cat ttc acc gac cca gct    5490
   Asn Tyr Asn Leu Phe Val Met Asp Glu Ala His Phe Thr Asp Pro Ala
           1785                1790                1795
   agt atc gca gca aga ggt tac att tcc aca aag gtc gag cta ggg gag    5538
   Ser Ile Ala Ala Arg Gly Tyr Ile Ser Thr Lys Val Glu Leu Gly Glu
       1800                1805                1810
   gcg gcg gca ata ttc atg aca gcc acc cca cca ggc act tca gat cca    5586
   Ala Ala Ala Ile Phe Met Thr Ala Thr Pro Pro Gly Thr Ser Asp Pro
   1815                1820                1825                1830
   ttc cca gag tcc aat tca cca att tcc gac tta cag act gag atc ccg    5634
   Phe Pro Glu Ser Asn Ser Pro Ile Ser Asp Leu Gln Thr Glu Ile Pro
                   1835                1840                1845
   gat cga gct tgg aac tct gga tac gaa tgg atc aca gaa tac acc ggg    5682
   Asp Arg Ala Trp Asn Ser Gly Tyr Glu Trp Ile Thr Glu Tyr Thr Gly
               1850                1855                1860
   aag acg gtt tgg ttt gtg cct agt gtc aag atg ggg aat gag att gcc    5730
   Lys Thr Val Trp Phe Val Pro Ser Val Lys Met Gly Asn Glu Ile Ala
           1865                1870                1875
   ctt tgc cta caa cgt gct gga aag aaa gta gtc caa ttg aac aga aag    5778
   Leu Cys Leu Gln Arg Ala Gly Lys Lys Val Val Gln Leu Asn Arg Lys
       1880                1885                1890
   tcg tac gag acg gag tac cca aaa tgt aag aac gat gat tgg gac ttt    5826
   Ser Tyr Glu Thr Glu Tyr Pro Lys Cys Lys Asn Asp Asp Trp Asp Phe
   1895                1900                1905                1910
   gtt atc aca aca gac ata tct gaa atg ggg gct aac ttc aag gcg agc    5874
   Val Ile Thr Thr Asp Ile Ser Glu Met Gly Ala Asn Phe Lys Ala Ser
                   1915                1920                1925
   agg gtg att gac agc cgg aag agt gtg aaa cca acc atc ata aca gaa    5922
   Arg Val Ile Asp Ser Arg Lys Ser Val Lys Pro Thr Ile Ile Thr Glu
               1930                1935                1940
   gga gaa ggg aga gtg atc ctg gga gaa cca tct gca gtg aca gca gct    5970
   Gly Glu Gly Arg Val Ile Leu Gly Glu Pro Ser Ala Val Thr Ala Ala
           1945                1950                1955
   agt gcc gcc cag aga cgt gga cgt atc ggt aga aat ccg tcg caa gtt    6018
   Ser Ala Ala Gln Arg Arg Gly Arg Ile Gly Arg Asn Pro Ser Gln Val
       1960                1965                1970
   ggt gat gag tac tgt tat ggg ggg cac acg aat gaa gac gac tcg aac    6066
   Gly Asp Glu Tyr Cys Tyr Gly Gly His Thr Asn Glu Asp Asp Ser Asn
   1975                1980                1985                1990
   ttc gcc cat tgg act gag gca cga atc atg ccg gac aac atc aac atg    6114
   Phe Ala His Trp Thr Glu Ala Arg Ile Met Pro Asp Asn Ile Asn Met
                   1995                2000                2005
   cca aac gga ctg atc gct caa ttc tac caa cca gag cgt gag aag gta    6162
   Pro Asn Gly Leu Ile Ala Gln Phe Tyr Gln Pro Glu Arg Glu Lys Val
               2010                2015                2020
   tat acc atg gag ggg gaa tac cgg ctc aga gga gaa gag agg aaa aac    6210
   Tyr Thr Met Glu Gly Glu Tyr Arg Leu Arg Gly Glu Glu Arg Lys Asn
           2025                2030                2035
   ttt ctg gaa ctg ttg agg act gca gat ctg cca gtt tgg ctg gct tac    6258
   Phe Leu Glu Leu Leu Arg Thr Ala Asp Leu Pro Val Trp Leu Ala Tyr
       2040                2045                2050
   aag gtt gca gcg gct gga gtg tca tac cac gac cgg agg tgg tgc ttt    6306
   Lys Val Ala Ala Ala Gly Val Ser Tyr His Asp Arg Arg Trp Cys Phe
   2055                2060                2065                2070
   gat ggt cct agg aca aac aca att tta gaa gac aac aac gaa gtg gaa    6354
   Asp Gly Pro Arg Thr Asn Thr Ile Leu Glu Asp Asn Asn Glu Val Glu
                   2075                2080                2085
   gtc atc acg aag ctt ggt gaa agg aag att ctg agg ccg cgc tgg att    6402
   Val Ile Thr Lys Leu Gly Glu Arg Lys Ile Leu Arg Pro Arg Trp Ile
               2090                2095                2100
   gac gcc agg gtg tac tcg gat cac cag gca cta aag gcg ttc aag gac    6450
   Asp Ala Arg Val Tyr Ser Asp His Gln Ala Leu Lys Ala Phe Lys Asp
           2105                2110                2115
   ttc gcc tcg gga aaa cgt tct cag ata ggg ctc att gag gtt ctg gga    6498
   Phe Ala Ser Gly Lys Arg Ser Gln Ile Gly Leu Ile Glu Val Leu Gly
       2120                2125                2130
   aag atg cct gag cac ttc atg ggg aag aca tgg gaa gca ctt gac acc    6546
   Lys Met Pro Glu His Phe Met Gly Lys Thr Trp Glu Ala Leu Asp Thr
   2135                2140                2145                2150
   atg tac gtt gtg gcc act gca gag aaa gga gga aga gct cac aga atg    6594
   Met Tyr Val Val Ala Thr Ala Glu Lys Gly Gly Arg Ala His Arg Met
                   2155                2160                2165
   gcc ctg gag gaa ctg cca gat gct ctt cag aca att gcc ttg att gcc    6642
   Ala Leu Glu Glu Leu Pro Asp Ala Leu Gln Thr Ile Ala Leu Ile Ala
               2170                2175                2180
   tta ttg agt gtg atg acc atg gga gta ttc ttc ctc ctc atg cag cgg    6690
   Leu Leu Ser Val Met Thr Met Gly Val Phe Phe Leu Leu Met Gln Arg
           2185                2190                2195
   aag ggc att gga aag ata ggt ttg gga ggc gct gtc ttg gga gtc gcg    6738
   Lys Gly Ile Gly Lys Ile Gly Leu Gly Gly Ala Val Leu Gly Val Ala
       2200                2205                2210
   acc ttt ttc tgt tgg atg gct gaa gtt cca gga acg aag atc gcc gga    6786
   Thr Phe Phe Cys Trp Met Ala Glu Val Pro Gly Thr Lys Ile Ala Gly
   2215                2220                2225                2230
   atg ttg ctg ctc tcc ctt ctc ttg atg att gtg cta att cct gag cca 6834
   Met Leu Leu Leu Ser Leu Leu Leu Met Ile Val Leu Ile Pro Glu Pro
   2235 2240 2245
   gag aag caa cgt tcg cag aca gac aac cag cta gcc gtg ttc ctg att 6882
   Glu Lys Gln Arg Ser Gln Thr Asp Asn Gln Leu Ala Val Phe Leu Ile
   2250 2255 2260
   tgt gtc atg acc ctt gtg agc gca gtg gca gcc aac gag atg ggt tgg 6930
   Cys Val Met Thr Leu Val Ser Ala Val Ala Ala Asn Glu Met Gly Trp
   2265 2270 2275
   cta gac aag acc aag agt gac ata agc agt ttg ttt ggg caa aga att 6978
   Leu Asp Lys Thr Lys Ser Asp Ile Ser Ser Leu Phe Gly Gln Arg Ile
   2280 2285 2290
   gag gtc aag gag aat ttc agc atg gga gag ttt ctt ctg gac ttg agg 7026
   Glu Val Lys Glu Asn Phe Ser Met Gly Glu Phe Leu Leu Asp Leu Arg
   2295 2300 2305 2310
   ccg gca aca gcc tgg tca ctg tac gct gtg aca aca gcg gtc ctc act 7074
   Pro Ala Thr Ala Trp Ser Leu Tyr Ala Val Thr Thr Ala Val Leu Thr
   2315 2320 2325
   cca ctg cta aag cat ttg atc acg tca gat tac atc aac acc tca ttg 7122
   Pro Leu Leu Lys His Leu Ile Thr Ser Asp Tyr Ile Asn Thr Ser Leu
   2330 2335 2340
   acc tca ata aac gtt cag gca agt gca cta ttc aca ctc gcg cga ggc 7170
   Thr Ser Ile Asn Val Gln Ala Ser Ala Leu Phe Thr Leu Ala Arg Gly
   2345 2350 2355
   ttc ccc ttc gtc gat gtt gga gtg tcg gct ctc ctg cta gca gcc gga 7218
   Phe Pro Phe Val Asp Val Gly Val Ser Ala Leu Leu Leu Ala Ala Gly
   2360 2365 2370
   tgc tgg gga caa gtc acc ctc acc gtt acg gta aca gcg gca aca ctc 7266
   Cys Trp Gly Gln Val Thr Leu Thr Val Thr Val Thr Ala Ala Thr Leu
   2375 2380 2385 2390
   ctt ttt tgc cac tat gcc tac atg gtt ccc ggt tgg caa gct gag gca 7314
   Leu Phe Cys His Tyr Ala Tyr Met Val Pro Gly Trp Gln Ala Glu Ala
   2395 2400 2405
   atg cgc tca gcc cag cgg cgg aca gcg gcc gga atc atg aaa aac gct 7362
   Met Arg Ser Ala Gln Arg Arg Thr Ala Ala Gly Ile Met Lys Asn Ala
   2410 2415 2420
   gta gtg gat ggc atc gtg gcc acg gac gtc cca gaa tta gag cgc acc 7410
   Val Val Asp Gly Ile Val Ala Thr Asp Val Pro Glu Leu Glu Arg Thr
   2425 2430 2435
   aca ccc atc atg cag aag aaa gtt gga cag atc atg ctg atc ttg gtg 7458
   Thr Pro Ile Met Gln Lys Lys Val Gly Gln Ile Met Leu Ile Leu Val
   2440 2445 2450
   tct cta gct gca gta gta gtg aac ccg tct gtg aag aca gta cga gaa 7506
   Ser Leu Ala Ala Val Val Val Asn Pro Ser Val Lys Thr Val Arg Glu
   2455 2460 2465 2470
   gcc gga att ttg atc acg gcc gca gcg gtg acg ctt tgg gag aat gga   7554
   Ala Gly Ile Leu Ile Thr Ala Ala Ala Val Thr Leu Trp Glu Asn Gly
               2475                2480                2485
   gca agc tct gtt tgg aac gca aca act gcc atc gga ctc tgc cac atc   7602
   Ala Ser Ser Val Trp Asn Ala Thr Thr Ala Ile Gly Leu Cys His Ile
           2490                2495                2500
   atg cgt ggg ggt tgg ttg tca tgt cta tcc ata aca tgg aca ctc ata   7650
   Met Arg Gly Gly Trp Leu Ser Cys Leu Ser Ile Thr Trp Thr Leu Ile
       2505                2510                2515
   aag aac atg gaa aaa cca gga cta aaa aga ggt ggg gca aaa gga cgc   7698
   Lys Asn Met Glu Lys Pro Gly Leu Lys Arg Gly Gly Ala Lys Gly Arg
   2520                2525                2530
   acc ttg gga gag gtt tgg aaa gaa aga ctc aac cag atg aca aaa gaa   7746
   Thr Leu Gly Glu Val Trp Lys Glu Arg Leu Asn Gln Met Thr Lys Glu
   2535                2540                2545                2550
   gag ttc act agg tac cgc aaa gag gcc atc atc gaa gtc gat cgc tca   7794
   Glu Phe Thr Arg Tyr Arg Lys Glu Ala Ile Ile Glu Val Asp Arg Ser
               2555                2560                2565
   gcg gca aaa cac gcc agg aaa gaa ggc aat gtc act gga ggg cat tca   7842
   Ala Ala Lys His Ala Arg Lys Glu Gly Asn Val Thr Gly Gly His Ser
           2570                2575                2580
   gtc tct agg ggc aca gca aaa ctg aga tgg ctg gtc gaa cgg agg ttt   7890
   Val Ser Arg Gly Thr Ala Lys Leu Arg Trp Leu Val Glu Arg Arg Phe
       2585                2590                2595
   ctc gaa ccg gtc gga aaa gtg att gac ctt gga tgt gga aga ggc ggt   7938
   Leu Glu Pro Val Gly Lys Val Ile Asp Leu Gly Cys Gly Arg Gly Gly
   2600                2605                2610
   tgg tgt tac tat atg gca acc caa aaa aga gtc caa gaa gtc aga ggg   7986
   Trp Cys Tyr Tyr Met Ala Thr Gln Lys Arg Val Gln Glu Val Arg Gly
   2615                2620                2625                2630
   tac aca aag ggc ggt ccc gga cat gaa gag ccc caa cta gtg caa agt   8034
   Tyr Thr Lys Gly Gly Pro Gly His Glu Glu Pro Gln Leu Val Gln Ser
               2635                2640                2645
   tat gga tgg aac att gtc acc atg aag agt gga gtg gat gtg ttc tac   8082
   Tyr Gly Trp Asn Ile Val Thr Met Lys Ser Gly Val Asp Val Phe Tyr
           2650                2655                2660
   aga cct tct gag tgt tgt gac acc ctc ctt tgt gac atc gga gag tcc   8130
   Arg Pro Ser Glu Cys Cys Asp Thr Leu Leu Cys Asp Ile Gly Glu Ser
       2665                2670                2675
   tcg tca agt gct gag gtt gaa gag cat agg acg att cgt gtc ctt gaa   8178
   Ser Ser Ser Ala Glu Val Glu Glu His Arg Thr Ile Arg Val Leu Glu
   2680                2685                2690
   atg gtt gag gac tgg ctg cac cga ggg cca agg gaa ttt tgc gtg aag   8226
   Met Val Glu Asp Trp Leu His Arg Gly Pro Arg Glu Phe Cys Val Lys
   2695                2700                2705                2710
   gtg ctc tgc ccc tac atg ccg aaa gtc ata gag aag atg gag ctg ctc    8274
   Val Leu Cys Pro Tyr Met Pro Lys Val Ile Glu Lys Met Glu Leu Leu
                   2715                2720                2725
   caa cgc cgg tat ggg ggg gga ctg gtc aga aac cca ctc tca cgg aat    8322
   Gln Arg Arg Tyr Gly Gly Gly Leu Val Arg Asn Pro Leu Ser Arg Asn
               2730                2735                2740
   tcc acg cac gag atg tat tgg gtg agt cga gct tca ggc aat gtg gta    8370
   Ser Thr His Glu Met Tyr Trp Val Ser Arg Ala Ser Gly Asn Val Val
           2745                2750                2755
   cat tca gtg aat atg acc agc cag gtg ctc cta gga aga atg gaa aaa    8418
   His Ser Val Asn Met Thr Ser Gln Val Leu Leu Gly Arg Met Glu Lys
       2760                2765                2770
   agg acc tgg aag gga ccc caa tac gag gaa gat gta aac ttg gga agc    8466
   Arg Thr Trp Lys Gly Pro Gln Tyr Glu Glu Asp Val Asn Leu Gly Ser
   2775                2780                2785                2790
   gga acc agg gcg gtg gga aaa ccc ctg ctc aac tca gac acc agt aaa    8514
   Gly Thr Arg Ala Val Gly Lys Pro Leu Leu Asn Ser Asp Thr Ser Lys
                   2795                2800                2805
   atc aac aac agg att gaa cga ctc agg cgt gag tac agt tcg acg tgg    8562
   Ile Asn Asn Arg Ile Glu Arg Leu Arg Arg Glu Tyr Ser Ser Thr Trp
               2810                2815                2820
   cac cac gat gag aac cac cca tat aga acc tgg aac tat cac ggc agt    8610
   His His Asp Glu Asn His Pro Tyr Arg Thr Trp Asn Tyr His Gly Ser
           2825                2830                2835
   tat gat gtg aag ccc aca ggc tcc gcc agt tcg ctg gtc aat gga gtg    8658
   Tyr Asp Val Lys Pro Thr Gly Ser Ala Ser Ser Leu Val Asn Gly Val
       2840                2845                2850
   gtc agg ctc ctc tca aaa cca tgg gac acc atc acg aat gtt acc acc    8706
   Val Arg Leu Leu Ser Lys Pro Trp Asp Thr Ile Thr Asn Val Thr Thr
   2855                2860                2865                2870
   atg gcc atg act gac act act ccc ttc ggg cag cag cga gtg ttc aaa    8754
   Met Ala Met Thr Asp Thr Thr Pro Phe Gly Gln Gln Arg Val Phe Lys
                   2875                2880                2885
   gag aag gtg gac acg aaa gct cct gaa ccg cca gaa gga gcg aag tac    8802
   Glu Lys Val Asp Thr Lys Ala Pro Glu Pro Pro Glu Gly Ala Lys Tyr
               2890                2895                2900
   gtg ctc aac gag acc acc aac tgg ttg tgg gcg ttt ttg gcc aga gaa    8850
   Val Leu Asn Glu Thr Thr Asn Trp Leu Trp Ala Phe Leu Ala Arg Glu
           2905                2910                2915
   aaa cgt ccc aga atg tgc tct cga gag gaa ttc ata aga aag gtc aac    8898
   Lys Arg Pro Arg Met Cys Ser Arg Glu Glu Phe Ile Arg Lys Val Asn
       2920                2925                2930
   agc aat gca gct ttg ggt gcc atg ttt gaa gag cag aat caa tgg agg    8946
   Ser Asn Ala Ala Leu Gly Ala Met Phe Glu Glu Gln Asn Gln Trp Arg
   2935                2940                2945                2950
   agc gcc aga gaa gca gtt gaa gat cca aaa ttt tgg gag atg gtg gat      8994
   Ser Ala Arg Glu Ala Val Glu Asp Pro Lys Phe Trp Glu Met Val Asp
                   2955                2960                2965
   gag gag cgc gag gca cat ctg cgg ggg gaa tgt cac act tgc att tac      9042
   Glu Glu Arg Glu Ala His Leu Arg Gly Glu Cys His Thr Cys Ile Tyr
               2970                2975                2980
   aac atg atg gga aag aga gag aaa aaa ccc gga gag ttc gga aag gcc      9090
   Asn Met Met Gly Lys Arg Glu Lys Lys Pro Gly Glu Phe Gly Lys Ala
           2985                2990                2995
   aag gga agc aga gcc att tgg ttc atg tgg ctc gga gct cgc ttt ctg      9138
   Lys Gly Ser Arg Ala Ile Trp Phe Met Trp Leu Gly Ala Arg Phe Leu
       3000                3005                3010
   gag ttc gag gct ctg ggt ttt ctc aat gaa gac cac tgg ctt gga aga      9186
   Glu Phe Glu Ala Leu Gly Phe Leu Asn Glu Asp His Trp Leu Gly Arg
   3015                3020                3025                3030
   aag aac tca gga gga ggt gtc gag ggc ttg ggc ctc caa aaa ctg ggt      9234
   Lys Asn Ser Gly Gly Gly Val Glu Gly Leu Gly Leu Gln Lys Leu Gly
                   3035                3040                3045
   tac atc ctg cgt gaa gtt ggc acc cgg cct ggg ggc aag atc tat gct      9282
   Tyr Ile Leu Arg Glu Val Gly Thr Arg Pro Gly Gly Lys Ile Tyr Ala
               3050                3055                3060
   gat gac aca gct ggc tgg gac acc cgc atc acg aga gct gac ttg gaa      9330
   Asp Asp Thr Ala Gly Trp Asp Thr Arg Ile Thr Arg Ala Asp Leu Glu
           3065                3070                3075
   aat gaa gct aag gtg ctt gag ctg ctt gat ggg gaa cat cgg cgt ctt      9378
   Asn Glu Ala Lys Val Leu Glu Leu Leu Asp Gly Glu His Arg Arg Leu
       3080                3085                3090
   gcc agg gcc atc att gag ctc acc tat cgt cac aaa gtt gtg aaa gtg      9426
   Ala Arg Ala Ile Ile Glu Leu Thr Tyr Arg His Lys Val Val Lys Val
   3095                3100                3105                3110
   atg cgc ccg gct gct gat gga aga acc gtc atg gat gtt atc tcc aga      9474
   Met Arg Pro Ala Ala Asp Gly Arg Thr Val Met Asp Val Ile Ser Arg
                   3115                3120                3125
   gaa gat cag agg ggg agt gga caa gtt gtc acc tac gcc cta aac act      9522
   Glu Asp Gln Arg Gly Ser Gly Gln Val Val Thr Tyr Ala Leu Asn Thr
               3130                3135                3140
   ttc acc aac ctg gcc gtc cag ctg gtg agg atg atg gaa ggg gaa gga      9570
   Phe Thr Asn Leu Ala Val Gln Leu Val Arg Met Met Glu Gly Glu Gly
           3145                3150                3155
   gtg att ggc cca gat gat gtg gag aaa ctc aca aaa ggg aaa gga ccc      9618
   Val Ile Gly Pro Asp Asp Val Glu Lys Leu Thr Lys Gly Lys Gly Pro
       3160                3165                3170
   aaa gtc agg acc tgg ctg ttt gag aat ggg gaa gaa aga ctc agc cgc      9666
   Lys Val Arg Thr Trp Leu Phe Glu Asn Gly Glu Glu Arg Leu Ser Arg
   3175                3180                3185                3190
   atg gct gtc agt gga gat gac tgt gtg gta aag ccc ctg gac gat cgc    9714
   Met Ala Val Ser Gly Asp Asp Cys Val Val Lys Pro Leu Asp Asp Arg
                   3195                3200                3205
   ttt gcc acc tcg ctc cac ttc ctc aat gct atg tca aag gtt cgc aaa    9762
   Phe Ala Thr Ser Leu His Phe Leu Asn Ala Met Ser Lys Val Arg Lys
               3210                3215                3220
   gac atc caa gag tgg aaa ccg tca act gga tgg tat gat tgg cag cag    9810
   Asp Ile Gln Glu Trp Lys Pro Ser Thr Gly Trp Tyr Asp Trp Gln Gln
           3225                3230                3235
   gtt cca ttt tgc tca aac cat ttc act gaa ttg atc atg aaa gat gga    9858
   Val Pro Phe Cys Ser Asn His Phe Thr Glu Leu Ile Met Lys Asp Gly
       3240                3245                3250
   aga aca ctg gtg gtt cca tgc cga gga cag gat gaa ttg gta ggc aga    9906
   Arg Thr Leu Val Val Pro Cys Arg Gly Gln Asp Glu Leu Val Gly Arg
   3255                3260                3265                3270
   gct cgc ata tct cca ggg gcc gga tgg aac gtc cgc gac act gct tgt    9954
   Ala Arg Ile Ser Pro Gly Ala Gly Trp Asn Val Arg Asp Thr Ala Cys
                   3275                3280                3285
   ctg gct aag tct tat gcc cag atg tgg ctg ctt ctg tac ttc cac aga    10002
   Leu Ala Lys Ser Tyr Ala Gln Met Trp Leu Leu Leu Tyr Phe His Arg
               3290                3295                3300
   aga gac ctg cgg ctc atg gcc aac gcc att tgc tcc gct gtc cct gtg    10050
   Arg Asp Leu Arg Leu Met Ala Asn Ala Ile Cys Ser Ala Val Pro Val
           3305                3310                3315
   aat tgg gtc cct acc gga aga acc acg tgg tcc atc cat gca gga gga    10098
   Asn Trp Val Pro Thr Gly Arg Thr Thr Trp Ser Ile His Ala Gly Gly
       3320                3325                3330
   gag tgg atg aca aca gag gac atg ttg gag gtc tgg aac cgt gtt tgg    10146
   Glu Trp Met Thr Thr Glu Asp Met Leu Glu Val Trp Asn Arg Val Trp
   3335                3340                3345                3350
   ata gag gag aat gaa tgg atg gaa gac aaa acc cca gtg gag aaa tgg    10194
   Ile Glu Glu Asn Glu Trp Met Glu Asp Lys Thr Pro Val Glu Lys Trp
                   3355                3360                3365
   agt gac gtc cca tat tca gga aaa cga gag gac atc tgg tgt ggc agc    10242
   Ser Asp Val Pro Tyr Ser Gly Lys Arg Glu Asp Ile Trp Cys Gly Ser
               3370                3375                3380
   ctg att ggc aca aga gcc cga gcc acg tgg gca gaa aac atc cag gtg    10290
   Leu Ile Gly Thr Arg Ala Arg Ala Thr Trp Ala Glu Asn Ile Gln Val
           3385                3390                3395
   gct atc aac caa gtc aga gca atc atc gga gat gag aag tat gtg gat    10338
   Ala Ile Asn Gln Val Arg Ala Ile Ile Gly Asp Glu Lys Tyr Val Asp
       3400                3405                3410
   tac atg agt tca cta aag aga tat gaa gac aca act ttg gtt gag gac    10386
   Tyr Met Ser Ser Leu Lys Arg Tyr Glu Asp Thr Thr Leu Val Glu Asp
   3415                3420                3425                3430
   aca gta ctg tagatattta atcaattgta aatagacaat ataagtatgc 10435
   Thr Val Leu
   ataaaagtgt agttttatag tagtatttag tggtgttagt gtaaatagtt aagaaaattt 10495
   tgaggagaaa gtcaggccgg gaagttcccg ccaccggaag ttgagtagac ggtgctgcct 10555
   gcgactcaac cccaggagga ctgggtgaac aaagccgcga agtgatccat gtaagccctc 10615
   agaaccgtct cggaaggagg accccacatg ttgtaacttc aaagcccaat gtcagaccac 10675
   gctacggcgt gctactctgc ggagagtgca gtctgcgata gtgccccagg aggactgggt 10735
   taacaaaggc aaaccaacgc cccacgcggc cctagccccg gtaatggtgt taaccagggc 10795
   gaaaggacta gaggttagag gagaccccgc ggtttaaagt gcacggccca gcctgactga 10855
   agctgtaggt caggggaagg actagaggtt agtggagacc ccgtgccaca aaacaccaca 10915
   acaaaacagc atattgacac ctgggataga ctaggagatc ttctgctctg cacaaccagc 10975
   cacacggcac agtgcgccga caatggtggc tggtggtgcg agaacacagg atct 11029
<210> 2
   <211> 3433
   <212> PRT
   <213> Flavivirus sp.
<400> 2
   Met Ser Lys Lys Pro Gly Gly Pro Gly Lys Ser Arg Ala Val Asn Met
    1               5                  10                  15
   Leu Lys Arg Gly Met Pro Arg Val Leu Ser Leu Ile Gly Leu Lys Arg
               20                  25                  30
   Ala Met Leu Ser Leu Ile Asp Gly Lys Gly Pro Ile Arg Phe Val Leu
           35                  40                  45
   Ala Leu Leu Ala Phe Phe Arg Phe Thr Ala Ile Ala Pro Thr Arg Ala
       50                  55                  60
   Val Leu Asp Arg Trp Arg Gly Val Asn Lys Gln Thr Ala Met Lys His
   65                  70                  75                  80
   Leu Leu Ser Phe Lys Lys Glu Leu Gly Thr Leu Thr Ser Ala Ile Asn
                   85                  90                  95
   Arg Arg Ser Ser Lys Gln Lys Lys Arg Gly Gly Lys Thr Gly Ile Ala
               100                 105                 110
   Val Met Ile Gly Leu Ile Ala Ser Val Gly Ala Val Thr Leu Ser Asn
           115                 120                 125
   Phe Gln Gly Lys Val Met Met Thr Val Asn Ala Thr Asp Val Thr Asp
       130                 135                 140
   Val Ile Thr Ile Pro Thr Ala Ala Gly Lys Asn Leu Cys Ile Val Arg
   145                 150                 155                 160
   Ala Met Asp Val Gly Tyr Met Cys Asp Asp Thr Ile Thr Tyr Glu Cys
                   165                 170                 175
   Pro Val Leu Ser Ala Gly Asn Asp Pro Glu Asp Ile Asp Cys Trp Cys
               180                 185                 190
   Thr Lys Ser Ala Val Tyr Val Arg Tyr Gly Arg Cys Thr Lys Thr Arg
           195                 200                 205
   His Ser Arg Arg Ser Arg Arg Ser Leu Thr Val Gln Thr His Gly Glu
       210                 215                 220
   Ser Thr Leu Ala Asn Lys Lys Gly Ala Trp Met Asp Ser Thr Lys Ala
   225                 230                 235                 240
   Thr Arg Tyr Leu Val Lys Thr Glu Ser Trp Ile Leu Arg Asn Pro Gly
                   245                 250                 255
   Tyr Ala Leu Val Ala Ala Val Ile Gly Trp Met Leu Gly Ser Asn Thr
               260                 265                 270
   Met Gln Arg Val Val Phe Val Val Leu Leu Leu Leu Val Ala Pro Ala
           275                 280                 285
   Tyr Ser Phe Asn Cys Leu Gly Met Ser Asn Arg Asp Phe Leu Glu Gly
       290                 295                 300
   Val Ser Gly Ala Thr Trp Val Asp Leu Val Leu Glu Gly Asp Ser Cys
   305                 310                 315                 320
   Val Thr Ile Met Ser Lys Asp Lys Pro Thr Ile Asp Val Lys Met Met
                   325                 330                 335
   Asn Met Glu Ala Ala Asn Leu Ala Glu Val Arg Ser Tyr Cys Tyr Leu
               340                 345                 350
   Ala Thr Val Ser Asp Leu Ser Thr Lys Ala Ala Cys Pro Thr Met Gly
           355                 360                 365
   Glu Ala His Asn Asp Lys Arg Ala Asp Pro Ala Phe Val Cys Arg Gln
       370                 375                 380
   Gly Val Val Asp Arg Gly Trp Gly Asn Gly Cys Gly Leu Phe Gly Lys
   385                 390                 395                 400
   Gly Ser Ile Asp Thr Cys Ala Lys Phe Ala Cys Ser Thr Lys Ala Ile
                   405                 410                 415
   Gly Arg Thr Ile Leu Lys Glu Asn Ile Lys Tyr Glu Val Ala Ile Phe
               420                 425                 430
   Val His Gly Pro Thr Thr Val Glu Ser His Gly Asn Tyr Ser Thr Gln
           435                 440                 445
   Val Gly Ala Thr Gln Ala Gly Arg Phe Ser Ile Thr Pro Ala Ala Pro
       450                 455                 460
   Ser Tyr Thr Leu Lys Leu Gly Glu Tyr Gly Glu Val Thr Val Asp Cys
   465                 470                 475                 480
   Glu Pro Arg Ser Gly Ile Asp Thr Asn Ala Tyr Tyr Val Met Thr Val
   485 490 495
   Gly Thr Lys Thr Phe Leu Val His Arg Glu Trp Phe Met Asp Leu Asn
   500 505 510
   Leu Pro Trp Ser Ser Ala Gly Ser Thr Val Trp Arg Asn Arg Glu Thr
   515 520 525
   Leu Met Glu Phe Glu Glu Pro His Ala Thr Lys Gln Ser Val Ile Ala
   530 535 540
   Leu Gly Ser Gln Glu Gly Ala Leu His Gln Ala Leu Ala Gly Ala Ile
   545 550 555 560
   Pro Val Glu Phe Ser Ser Asn Thr Val Lys Leu Thr Ser Gly His Leu
   565 570 575
   Lys Cys Arg Val Lys Met Glu Lys Leu Gln Leu Lys Gly Thr Thr Tyr
   580 585 590
   Gly Val Cys Ser Lys Ala Phe Lys Phe Leu Gly Thr Pro Ala Asp Thr
   595 600 605
   Gly His Gly Thr Val Val Leu Glu Leu Gln Tyr Thr Gly Thr Asp Gly
   610 615 620
   Pro Cys Lys Val Pro Ile Ser Ser Val Ala Ser Leu Asn Asp Leu Thr
   625 630 635 640
   Pro Val Gly Arg Leu Val Thr Val Asn Pro Phe Val Ser Val Ala Thr
   645 650 655
   Ala Asn Ala Lys Val Leu Ile Glu Leu Glu Pro Pro Phe Gly Asp Ser
   660 665 670
   Tyr Ile Val Val Gly Arg Gly Glu Gln Gln Ile Asn His His Trp His
   675 680 685
   Lys Ser Gly Ser Ser Ile Gly Lys Ala Phe Thr Thr Thr Leu Lys Gly
   690 695 700
   Ala Gln Arg Leu Ala Ala Leu Gly Asp Thr Ala Trp Asp Phe Gly Ser
   705 710 715 720
   Val Gly Gly Val Phe Thr Ser Val Gly Lys Ala Val His Gln Val Phe
   725 730 735
   Gly Gly Ala Phe Arg Ser Leu Phe Gly Gly Met Ser Trp Ile Thr Gln
   740 745 750
   Gly Leu Leu Gly Ala Leu Leu Leu Trp Met Gly Ile Asn Ala Arg Asp
   755 760 765
   Arg Ser Ile Ala Leu Thr Phe Leu Ala Val Gly Gly Val Leu Leu Phe
   770 775 780
   Leu Ser Val Asn Val His Ala Asp Thr Gly Cys Ala Ile Asp Ile Ser
   785 790 795 800
   Arg Gln Glu Leu Arg Cys Gly Asn Gly Val Phe Ile His Asn Asp Val
   805 810 815
   Glu Ala Trp Met Asp Arg Tyr Lys Tyr Tyr Pro Glu Thr Pro Gln Gly
   820 825 830
   Leu Ala Lys Ile Ile Gln Lys Ala His Lys Glu Gly Val Cys Gly Leu
   835 840 845
   Arg Ser Val Ser Arg Leu Glu His Gln Met Trp Glu Ala Val Lys Asp
   850 855 860
   Glu Leu Asn Thr Leu Leu Lys Glu Asn Gly Val Asp Leu Ser Val Val
   865 870 875 880
   Val Glu Lys Gln Glu Gly Met Tyr Lys Ser Ala Pro Lys Arg Leu Thr
   885 890 895
   Ala Thr Thr Glu Lys Leu Glu Ile Gly Trp Lys Ala Trp Gly Lys Ser
   900 905 910
   Ile Leu Phe Ala Pro Glu Leu Ala Asn Asn Thr Phe Val Val Asp Gly
   915 920 925
   Pro Glu Thr Lys Glu Cys Pro Thr Gln Asn Arg Ala Trp Asn Ser Leu
   930 935 940
   Glu Val Glu Asp Phe Gly Phe Gly Leu Thr Ser Thr Arg Met Phe Leu
   945 950 955 960
   Lys Val Arg Glu Ser Asn Thr Thr Glu Cys Asp Ser Lys Ile Ile Gly
   965 970 975
   Thr Ala Val Lys Asn Asn Leu Ala Ile His Ser Asp Leu Ser Tyr Trp
   980 985 990
   Ile Glu Ser Arg Leu Asn Asp Thr Trp Lys Leu Glu Arg Ala Val Leu
   995 1000 1005
   Gly Glu Val Lys Ser Cys Thr Trp Pro Glu Thr His Thr Leu Trp Gly
   1010 1015 1020
   Asp Gly Ile Leu Glu Ser Asp Leu Ile Ile Pro Val Thr Leu Ala Gly
   025 1030 1035 1040
   Pro Arg Ser Asn His Asn Arg Arg Pro Gly Tyr Lys Thr Gln Asn Gln
   1045 1050 1055
   Gly Pro Trp Asp Glu Gly Arg Val Glu Ile Asp Phe Asp Tyr Cys Pro
   1060 1065 1070
   Gly Thr Thr Val Thr Leu Ser Glu Ser Cys Gly His Arg Gly Pro Ala
   1075 1080 1085
   Thr Arg Thr Thr Thr Glu Ser Gly Lys Leu Ile Thr Asp Trp Cys Cys
   1090 1095 1100
   Arg Ser Cys Thr Leu Pro Pro Leu Arg Tyr Gln Thr Asp Ser Gly Cys
   105 1110 1115 1120
   Trp Tyr Gly Met Glu Ile Arg Pro Gln Arg His Asp Glu Lys Thr Leu
   1125 1130 1135
   Val Gln Ser Gln Val Asn Ala Tyr Asn Ala Asp Met Ile Asp Pro Phe
   1140 1145 1150
   Gln Leu Gly Leu Leu Val Val Phe Leu Ala Thr Gln Glu Val Leu Arg
   1155 1160 1165
   Lys Arg Trp Thr Ala Lys Ile Ser Met Pro Ala Ile Leu Ile Ala Leu
   1170 1175 1180
   Leu Val Leu Val Phe Gly Gly Ile Thr Tyr Thr Asp Val Leu Arg Tyr
   185 1190 1195 1200
   Val Ile Leu Val Gly Ala Ala Phe Ala Glu Ser Asn Ser Gly Gly Asp
   1205 1210 1215
   Val Val His Leu Ala Leu Met Ala Thr Phe Lys Ile Gln Pro Val Phe
   1220 1225 1230
   Met Val Ala Ser Phe Leu Lys Ala Arg Trp Thr Asn Gln Glu Asn Ile
   1235 1240 1245
   Leu Leu Met Leu Ala Ala Val Phe Phe Gln Met Ala Tyr His Asp Ala
   1250 1255 1260
   Arg Gln Ile Leu Leu Trp Glu Ile Pro Asp Val Leu Asn Ser Leu Ala
   265 1270 1275 1280
   Val Ala Trp Met Ile Leu Arg Ala Ile Thr Phe Thr Thr Thr Ser Asn
   1285 1290 1295
   Val Val Val Pro Leu Leu Ala Leu Leu Thr Pro Arg Leu Arg Cys Leu
   1300 1305 1310
   Asn Leu Asp Val Tyr Arg Ile Leu Leu Leu Met Val Gly Ile Gly Ser
   1315 1320 1325
   Leu Ile Arg Glu Lys Arg Ser Ala Ala Ala Lys Lys Lys Gly Ala Ser
   1330 1335 1340
   Leu Leu Cys Leu Ala Leu Ala Ser Thr Gly Leu Phe Asn Pro Met Ile
   345 1350 1355 1360
   Leu Ala Ala Gly Leu Ile Ala Cys Asp Pro Asn Arg Lys Arg Gly Trp
   1365 1370 1375
   Pro Ala Thr Glu Val Met Thr Ala Val Gly Leu Met Phe Ala Ile Val
   1380 1385 1390
   Gly Gly Leu Ala Glu Leu Asp Ile Asp Ser Met Ala Ile Pro Met Thr
   1395 1400 1405
   Ile Ala Gly Leu Met Phe Ala Ala Phe Val Ile Ser Gly Lys Ser Thr
   1410 1415 1420
   Asp Met Trp Ile Glu Arg Thr Ala Asp Ile Ser Trp Glu Ser Asp Ala
   425 1430 1435 1440
   Glu Ile Thr Gly Ser Ser Glu Arg Val Asp Val Arg Leu Asp Asp Gly
   1445 1450 1455
   Glu Asn Phe Gln Leu Met Asn Asp Pro Gly Ala Pro Trp Lys Ile Trp
   1460 1465 1470
   Met Leu Arg Met Val Cys Leu Ala Ile Ser Ala Tyr Thr Pro Trp Ala
   1475 1480 1485
   Ile Leu Pro Ser Val Val Gly Phe Trp Ile Thr Leu Gln Tyr Thr Lys
   1490 1495 1500
   Arg Gly Gly Val Leu Trp Asp Thr Pro Ser Pro Lys Glu Tyr Lys Lys
   505 1510 1515 1520
   Gly Asp Thr Thr Thr Gly Val Tyr Arg Ile Met Thr Arg Gly Leu Leu
   1525 1530 1535
   Gly Ser Tyr Gln Ala Gly Ala Gly Val Met Val Glu Gly Val Phe His
   1540 1545 1550
   Thr Leu Trp His Thr Thr Lys Gly Ala Ala Leu Met Ser Gly Glu Gly
   1555 1560 1565
   Arg Leu Asp Pro Tyr Trp Gly Ser Val Lys Glu Asp Arg Leu Cys Tyr
   1570 1575 1580
   Gly Gly Pro Trp Lys Leu Gln His Lys Trp Asn Gly Gln Asp Glu Val
   585 1590 1595 1600
   Gln Met Ile Val Val Glu Pro Gly Lys Asn Val Lys Asn Val Gln Thr
   1605 1610 1615
   Lys Pro Gly Val Phe Lys Thr Pro Glu Gly Glu Ile Gly Ala Val Thr
   1620 1625 1630
   Leu Asp Phe Pro Thr Gly Thr Ser Gly Ser Pro Ile Val Asp Lys Asn
   1635 1640 1645
   Gly Asp Val Ile Gly Leu Tyr Gly Asn Gly Val Ile Met Pro Asn Gly
   1650 1655 1660
   Ser Tyr Ile Ser Ala Ile Val Gln Gly Glu Arg Met Asp Glu Pro Ile
   665 1670 1675 1680
   Pro Ala Gly Phe Glu Pro Glu Met Leu Arg Lys Lys Gln Ile Thr Val
   1685 1690 1695
   Leu Asp Leu His Pro Gly Ala Gly Lys Thr Arg Arg Ile Leu Pro Gln
   1700 1705 1710
   Ile Ile Lys Glu Ala Ile Asn Arg Arg Leu Arg Thr Ala Val Leu Ala
   1715 1720 1725
   Pro Thr Arg Val Val Ala Ala Glu Met Ala Glu Ala Leu Arg Gly Leu
   1730 1735 1740
   Pro Ile Arg Tyr Gln Thr Ser Ala Val Pro Arg Glu His Asn Gly Asn
   745 1750 1755 1760
   Glu Ile Val Asp Val Met Cys His Ala Thr Leu Thr His Arg Leu Met
   1765 1770 1775
   Ser Pro His Arg Val Pro Asn Tyr Asn Leu Phe Val Met Asp Glu Ala
   1780 1785 1790
   His Phe Thr Asp Pro Ala Ser Ile Ala Ala Arg Gly Tyr Ile Ser Thr
   1795 1800 1805
   Lys Val Glu Leu Gly Glu Ala Ala Ala Ile Phe Met Thr Ala Thr Pro
   1810 1815 1820
   Pro Gly Thr Ser Asp Pro Phe Pro Glu Ser Asn Ser Pro Ile Ser Asp
   825 1830 1835 1840
   Leu Gln Thr Glu Ile Pro Asp Arg Ala Trp Asn Ser Gly Tyr Glu Trp
   1845 1850 1855
   Ile Thr Glu Tyr Thr Gly Lys Thr Val Trp Phe Val Pro Ser Val Lys
   1860 1865 1870
   Met Gly Asn Glu Ile Ala Leu Cys Leu Gln Arg Ala Gly Lys Lys Val
   1875 1880 1885
   Val Gln Leu Asn Arg Lys Ser Tyr Glu Thr Glu Tyr Pro Lys Cys Lys
   1890 1895 1900
   Asn Asp Asp Trp Asp Phe Val Ile Thr Thr Asp Ile Ser Glu Met Gly
   905 1910 1915 1920
   Ala Asn Phe Lys Ala Ser Arg Val Ile Asp Ser Arg Lys Ser Val Lys
   1925 1930 1935
   Pro Thr Ile Ile Thr Glu Gly Glu Gly Arg Val Ile Leu Gly Glu Pro
   1940 1945 1950
   Ser Ala Val Thr Ala Ala Ser Ala Ala Gln Arg Arg Gly Arg Ile Gly
   1955 1960 1965
   Arg Asn Pro Ser Gln Val Gly Asp Glu Tyr Cys Tyr Gly Gly His Thr
   1970 1975 1980
   Asn Glu Asp Asp Ser Asn Phe Ala His Trp Thr Glu Ala Arg Ile Met
   985 1990 1995 2000
   Pro Asp Asn Ile Asn Met Pro Asn Gly Leu Ile Ala Gln Phe Tyr Gln
   2005 2010 2015
   Pro Glu Arg Glu Lys Val Tyr Thr Met Glu Gly Glu Tyr Arg Leu Arg
   2020 2025 2030
   Gly Glu Glu Arg Lys Asn Phe Leu Glu Leu Leu Arg Thr Ala Asp Leu
   2035 2040 2045
   Pro Val Trp Leu Ala Tyr Lys Val Ala Ala Ala Gly Val Ser Tyr His
   2050 2055 2060
   Asp Arg Arg Trp Cys Phe Asp Gly Pro Arg Thr Asn Thr Ile Leu Glu
   065 2070 2075 2080
   Asp Asn Asn Glu Val Glu Val Ile Thr Lys Leu Gly Glu Arg Lys Ile
   2085 2090 2095
   Leu Arg Pro Arg Trp Ile Asp Ala Arg Val Tyr Ser Asp His Gln Ala
   2100 2105 2110
   Leu Lys Ala Phe Lys Asp Phe Ala Ser Gly Lys Arg Ser Gln Ile Gly
   2115 2120 2125
   Leu Ile Glu Val Leu Gly Lys Met Pro Glu His Phe Met Gly Lys Thr
   2130 2135 2140
   Trp Glu Ala Leu Asp Thr Met Tyr Val Val Ala Thr Ala Glu Lys Gly
   145 2150 2155 2160
   Gly Arg Ala His Arg Met Ala Leu Glu Glu Leu Pro Asp Ala Leu Gln
   2165 2170 2175
   Thr Ile Ala Leu Ile Ala Leu Leu Ser Val Met Thr Met Gly Val Phe
   2180 2185 2190
   Phe Leu Leu Met Gln Arg Lys Gly Ile Gly Lys Ile Gly Leu Gly Gly
   2195 2200 2205
   Ala Val Leu Gly Val Ala Thr Phe Phe Cys Trp Met Ala Glu Val Pro
   2210 2215 2220
   Gly Thr Lys Ile Ala Gly Met Leu Leu Leu Ser Leu Leu Leu Met Ile
   225 2230 2235 2240
   Val Leu Ile Pro Glu Pro Glu Lys Gln Arg Ser Gln Thr Asp Asn Gln
   2245 2250 2255
   Leu Ala Val Phe Leu Ile Cys Val Met Thr Leu Val Ser Ala Val Ala
   2260 2265 2270
   Ala Asn Glu Met Gly Trp Leu Asp Lys Thr Lys Ser Asp Ile Ser Ser
   2275 2280 2285
   Leu Phe Gly Gln Arg Ile Glu Val Lys Glu Asn Phe Ser Met Gly Glu
   2290 2295 2300
   Phe Leu Leu Asp Leu Arg Pro Ala Thr Ala Trp Ser Leu Tyr Ala Val
   305 2310 2315 2320
   Thr Thr Ala Val Leu Thr Pro Leu Leu Lys His Leu Ile Thr Ser Asp
   2325 2330 2335
   Tyr Ile Asn Thr Ser Leu Thr Ser Ile Asn Val Gln Ala Ser Ala Leu
   2340 2345 2350
   Phe Thr Leu Ala Arg Gly Phe Pro Phe Val Asp Val Gly Val Ser Ala
   2355 2360 2365
   Leu Leu Leu Ala Ala Gly Cys Trp Gly Gln Val Thr Leu Thr Val Thr
   2370 2375 2380
   Val Thr Ala Ala Thr Leu Leu Phe Cys His Tyr Ala Tyr Met Val Pro
   385 2390 2395 2400
   Gly Trp Gln Ala Glu Ala Met Arg Ser Ala Gln Arg Arg Thr Ala Ala
   2405 2410 2415
   Gly Ile Met Lys Asn Ala Val Val Asp Gly Ile Val Ala Thr Asp Val
   2420 2425 2430
   Pro Glu Leu Glu Arg Thr Thr Pro Ile Met Gln Lys Lys Val Gly Gln
   2435 2440 2445
   Ile Met Leu Ile Leu Val Ser Leu Ala Ala Val Val Val Asn Pro Ser
   2450 2455 2460
   Val Lys Thr Val Arg Glu Ala Gly Ile Leu Ile Thr Ala Ala Ala Val
   465 2470 2475 2480
   Thr Leu Trp Glu Asn Gly Ala Ser Ser Val Trp Asn Ala Thr Thr Ala
   2485 2490 2495
   Ile Gly Leu Cys His Ile Met Arg Gly Gly Trp Leu Ser Cys Leu Ser
   2500 2505 2510
   Ile Thr Trp Thr Leu Ile Lys Asn Met Glu Lys Pro Gly Leu Lys Arg
   2515 2520 2525
   Gly Gly Ala Lys Gly Arg Thr Leu Gly Glu Val Trp Lys Glu Arg Leu
   2530 2535 2540
   Asn Gln Met Thr Lys Glu Glu Phe Thr Arg Tyr Arg Lys Glu Ala Ile
   545 2550 2555 2560
   Ile Glu Val Asp Arg Ser Ala Ala Lys His Ala Arg Lys Glu Gly Asn
   2565 2570 2575
   Val Thr Gly Gly His Ser Val Ser Arg Gly Thr Ala Lys Leu Arg Trp
   2580 2585 2590
   Leu Val Glu Arg Arg Phe Leu Glu Pro Val Gly Lys Val Ile Asp Leu
   2595 2600 2605
   Gly Cys Gly Arg Gly Gly Trp Cys Tyr Tyr Met Ala Thr Gln Lys Arg
   2610 2615 2620
   Val Gln Glu Val Arg Gly Tyr Thr Lys Gly Gly Pro Gly His Glu Glu
   625 2630 2635 2640
   Pro Gln Leu Val Gln Ser Tyr Gly Trp Asn Ile Val Thr Met Lys Ser
   2645 2650 2655
   Gly Val Asp Val Phe Tyr Arg Pro Ser Glu Cys Cys Asp Thr Leu Leu
   2660 2665 2670
   Cys Asp Ile Gly Glu Ser Ser Ser Ser Ala Glu Val Glu Glu His Arg
   2675 2680 2685
   Thr Ile Arg Val Leu Glu Met Val Glu Asp Trp Leu His Arg Gly Pro
   2690 2695 2700
   Arg Glu Phe Cys Val Lys Val Leu Cys Pro Tyr Met Pro Lys Val Ile
   705 2710 2715 2720
   Glu Lys Met Glu Leu Leu Gln Arg Arg Tyr Gly Gly Gly Leu Val Arg
   2725 2730 2735
   Asn Pro Leu Ser Arg Asn Ser Thr His Glu Met Tyr Trp Val Ser Arg
   2740 2745 2750
   Ala Ser Gly Asn Val Val His Ser Val Asn Met Thr Ser Gln Val Leu
   2755 2760 2765
   Leu Gly Arg Met Glu Lys Arg Thr Trp Lys Gly Pro Gln Tyr Glu Glu
   2770 2775 2780
   Asp Val Asn Leu Gly Ser Gly Thr Arg Ala Val Gly Lys Pro Leu Leu
   785 2790 2795 2800
   Asn Ser Asp Thr Ser Lys Ile Asn Asn Arg Ile Glu Arg Leu Arg Arg
   2805 2810 2815
   Glu Tyr Ser Ser Thr Trp His His Asp Glu Asn His Pro Tyr Arg Thr
   2820 2825 2830
   Trp Asn Tyr His Gly Ser Tyr Asp Val Lys Pro Thr Gly Ser Ala Ser
   2835 2840 2845
   Ser Leu Val Asn Gly Val Val Arg Leu Leu Ser Lys Pro Trp Asp Thr
   2850 2855 2860
   Ile Thr Asn Val Thr Thr Met Ala Met Thr Asp Thr Thr Pro Phe Gly
   865 2870 2875 2880
   Gln Gln Arg Val Phe Lys Glu Lys Val Asp Thr Lys Ala Pro Glu Pro
   2885 2890 2895
   Pro Glu Gly Ala Lys Tyr Val Leu Asn Glu Thr Thr Asn Trp Leu Trp
   2900 2905 2910
   Ala Phe Leu Ala Arg Glu Lys Arg Pro Arg Met Cys Ser Arg Glu Glu
   2915 2920 2925
   Phe Ile Arg Lys Val Asn Ser Asn Ala Ala Leu Gly Ala Met Phe Glu
   2930 2935 2940
   Glu Gln Asn Gln Trp Arg Ser Ala Arg Glu Ala Val Glu Asp Pro Lys
   945 2950 2955 2960
   Phe Trp Glu Met Val Asp Glu Glu Arg Glu Ala His Leu Arg Gly Glu
   2965 2970 2975
   Cys His Thr Cys Ile Tyr Asn Met Met Gly Lys Arg Glu Lys Lys Pro
   2980 2985 2990
   Gly Glu Phe Gly Lys Ala Lys Gly Ser Arg Ala Ile Trp Phe Met Trp
   2995 3000 3005
   Leu Gly Ala Arg Phe Leu Glu Phe Glu Ala Leu Gly Phe Leu Asn Glu
   3010 3015 3020
   Asp His Trp Leu Gly Arg Lys Asn Ser Gly Gly Gly Val Glu Gly Leu
   025 3030 3035 3040
   Gly Leu Gln Lys Leu Gly Tyr Ile Leu Arg Glu Val Gly Thr Arg Pro
               3045                3050                3055
   Gly Gly Lys Ile Tyr Ala Asp Asp Thr Ala Gly Trp Asp Thr Arg Ile
           3060                3065                3070
   Thr Arg Ala Asp Leu Glu Asn Glu Ala Lys Val Leu Glu Leu Leu Asp
       3075                3080                3085
   Gly Glu His Arg Arg Leu Ala Arg Ala Ile Ile Glu Leu Thr Tyr Arg
   3090                3095                3100
   His Lys Val Val Lys Val Met Arg Pro Ala Ala Asp Gly Arg Thr Val
   105                 3110                3115                3120
   Met Asp Val Ile Ser Arg Glu Asp Gln Arg Gly Ser Gly Gln Val Val
                   3125                3130                3135
   Thr Tyr Ala Leu Asn Thr Phe Thr Asn Leu Ala Val Gln Leu Val Arg
               3140                3145                3150
   Met Met Glu Gly Glu Gly Val Ile Gly Pro Asp Asp Val Glu Lys Leu
           3155                3160                3165
   Thr Lys Gly Lys Gly Pro Lys Val Arg Thr Trp Leu Phe Glu Asn Gly
       3170                3175                3180
   Glu Glu Arg Leu Ser Arg Met Ala Val Ser Gly Asp Asp Cys Val Val
   185                 3190                3195                3200
   Lys Pro Leu Asp Asp Arg Phe Ala Thr Ser Leu His Phe Leu Asn Ala
                   3205                3210                3215
   Met Ser Lys Val Arg Lys Asp Ile Gln Glu Trp Lys Pro Ser Thr Gly
               3220                3225                3230
   Trp Tyr Asp Trp Gln Gln Val Pro Phe Cys Ser Asn His Phe Thr Glu
           3235                3240                3245
   Leu Ile Met Lys Asp Gly Arg Thr Leu Val Val Pro Cys Arg Gly Gln
       3250                3255                3260
   Asp Glu Leu Val Gly Arg Ala Arg Ile Ser Pro Gly Ala Gly Trp Asn
   265                 3270                3275                3280
   Val Arg Asp Thr Ala Cys Leu Ala Lys Ser Tyr Ala Gln Met Trp Leu
                   3285                3290                3295
   Leu Leu Tyr Phe His Arg Arg Asp Leu Arg Leu Met Ala Asn Ala Ile
               3300                3305                3310
   Cys Ser Ala Val Pro Val Asn Trp Val Pro Thr Gly Arg Thr Thr Trp
           3315                3320                3325
   Ser Ile His Ala Gly Gly Glu Trp Met Thr Thr Glu Asp Met Leu Glu
       3330                3335                3340
   Val Trp Asn Arg Val Trp Ile Glu Glu Asn Glu Trp Met Glu Asp Lys
   345                 3350                3355                3360
   Thr Pro Val Glu Lys Trp Ser Asp Val Pro Tyr Ser Gly Lys Arg Glu
   3365 3370 3375
   Asp Ile Trp Cys Gly Ser Leu Ile Gly Thr Arg Ala Arg Ala Thr Trp
   3380 3385 3390
   Ala Glu Asn Ile Gln Val Ala Ile Asn Gln Val Arg Ala Ile Ile Gly
   3395 3400 3405
   Asp Glu Lys Tyr Val Asp Tyr Met Ser Ser Leu Lys Arg Tyr Glu Asp
   3410 3415 3420
   Thr Thr Leu Val Glu Asp Thr Val Leu
   425 3430
<210> 3
   <211> 37
   <212> ADN
   <213> Séquence artificielle
<220>
   <223> Description de la séquence artificielle:AMORCE OLIGONUCLEOTIDIQUE
<400> 3
   tagcacgaag aattcgatgt ctaagaaacc aggaggg 37
<210> 4
   <211> 50
   <212> ADN
   <213> Séquence artificielle
<220>
   <223> Description de la séquence artificielle:AMORCE OLIGONUCLEOTIDIQUE
<400> 4
   aagttagccc gggttaatgc tcctacgctg gcgatcaggc caatcaggac 50
<210> 5
   <211> 28
   <212> ADN
   <213> Séquence artificielle
<220>
   <223> Description de la séquence artificielle:amorce
<400> 5
   agtagttcgc ctgtgtgagc tgacaaac 28
<210> 6
   <211> 29
   <212> ADN
   <213> Séquence artificielle
<220>
   <223> Description de la séquence artificielle:amorce
<400> 6
   agatcctgtg ttctcgcacc accagccac 29
<210> 7
   <211> 21
   <212> ADN
   <213> Séquence artificielle
<220>
   <223> Description de la séquence artificielle:amorce
<400> 7
   ggatggatgctwggkagc aac 21
<210> 8
   <211> 19
   <212> ADN
   <213> Séquence artificielle
<220>
   <223> Description de la séquence artificielle:amorce
<400> 8
   ccatccaagc ctccacatc 19
<210> 9
   <211> 30
   <212> ADN
   <213> Séquence artificielle
<220>
   <223> Description de la séquence artificielle:amorce
<400> 9
   tggatgggat ccaatatgcg tgataggtcc 30
<210> 10
   <211> 39
   <212> ADN
   <213> Séquence artificielle
<220>
   <223> Description de la séquence artificielle:amorce
<400> 10
   aaaagggtca atggtaccag cattttaagc attcacgtt 39
<210> 11
   <211> 36
   <212> ADN
   <213> Séquence artificielle
<220>
   <223> Description de la séquence artificielle:amorce
<400> 11
   tagcacgaagaattcgatgtctaaaaaccaggaggg 36